# EUROPEAN PATENT SPECIFICATION

(11) **EP 3 824 855 B1**
(45) Date of publication and mention of the grant of the patent: **21.08.2024**
(21) Application number: 19211330.6
(22) Date of filing: 25.11.2019
(51) Int. Cl.: A61F 13/15, A61F 13/49, A61F 13/496, A61F 13/84, A61F 13/53

(54) **SWIM DIAPER**
SCHWIMMWINDEL
COUCHE DE BAIN

(43) Date of publication of application: 26.05.2021
(73) Proprietor: Drylock Technologies NV, 9240 Zele (BE)
(72) Inventor: VAN INGELGEM, Werner, 9420 Zele (BE)
(74) Representative: D'Halleweyn, Nele Veerle Trees Gertrudis

(56) References cited:
- EP-A1- 3 453 368
- US-A1- 2016 346 136

## Description

### TECHNICAL FIELD

The present invention pertains to the technical field of swim diapers, more preferably disposable swim pants, and to absorbent structures for use in such swim diapers. More specifically the present invention relates to a swim diaper comprising an absorbent core between a topsheet and a backsheet.

### BACKGROUND

Absorbent articles such as diapers, baby pants, adult incontinent garments and the like, typically comprise an absorbent core, positioned in between a liquid permeable or pervious, hydrophilic or semi hydrophilic topsheet and a liquid impermeable or impervious backsheet. The absorbent core comprises absorbent material that is able to absorb fluid and liquid bodily excretions of the user of the absorbent article. The absorbent material of the absorbent core may be an absorbent particulate polymer material which is dispersed in a matrix of cellulose fibers or fluff pulp in order to prevent the particulate material from aggregating.

Swim diapers are typically worn by babies, infants, toddlers and children in situations where it is expected that the diapers will be in continuous or frequent contact with water, for example in a swimming pool, in a bath or in sea. Absorbent cores of regular absorbent articles as described above are not suited to be used in swim diapers for various reasons.

Regular absorbent cores generally have a high absorbent capacity and the absorbent core may expand several times its weight and volume when used in wet environments. These increases may cause the absorbent article to deform and to sag in the crotch region as they become saturated with liquid. In addition, such a saturated absorbent core will become very heavy and this may impede the movements of the wearer both in the water, potentially causing a drowning hazard, and out of the water. Moreover, a heavy sagging diaper may cause skin irritation or even cuts or abrasions to the legs and/or waist of the wearer.

Furthermore, existing absorbent cores containing fluff pulp have a limited wet integrity, which leads to the shape and fit of the absorbent article being deformed when e.g. the absorbent article is being worn by a baby in a bath or a toddler in a pool. In other words, such fibrous materials may disintegrate in water and failure of such an absorbent core may result in loose fibrous material being introduced into the pool water. This may cause contamination of the water and may pose a possible health hazard for other children in the pool. In addition, when a diaper would fail in a pool, this might cause fecal waste to be introduced into the pool which would also cause contamination of the water. US 2016/346136 discloses a diaper.

### SUMMARY

It is an object of embodiments of the invention to provide a swim diaper with improved dry and wet integrity. Moreover, it is an object of embodiments of the invention to provide a swim diaper which is comfortable to wear in a dry and/or wet state. Furthermore it is an object of embodiments of the invention to provide a swim diaper which, if a fecal accident arises, contains the feces and prevents contamination of the surroundings.

According to an aspect of the invention there is provided a swim diaper comprising a liquid pervious topsheet, a liquid impervious backsheet, and an absorbent core positioned in between said topsheet and said backsheet. The absorbent core comprises a top core wrap sheet and a back core wrap sheet and fluff pulp between the top core wrap sheet and the back core wrap sheet. The absorbent core has a first and second longitudinal edge and a front and rear transverse edge. The absorbent core is substantially free of superabsorbent polymers. Seen in a longitudinal direction of the absorbent core, looking from the front transverse edge to the rear transverse edge, the absorbent core comprises subsequently a first, second, third and fourth zone. The absorbent core comprises a front part extending between the front edge and a transverse crotch line of the absorbent core, and a rear part extending between the rear edge and the transverse crotch line of the absorbent core. The first and second zones extend in the front part of the absorbent core and the third and fourth zone extend in the rear part. The absorbent core is provided with one or more reinforcement zones, where the top core wrap sheet is attached to the back core wrap sheet, extending at least partially in the second and/or third zone. The one or more reinforcement zones are arranged such that for any area in the second and/or third zone having a width of 50% of a width of the absorbent core and a length of 40% of a length of the absorbent core, at least one reinforcement zone is at least partially located in said area.

The second zone and third zone together extend over a length corresponding with at least 40% of the length of the absorbent core, preferably at least 50% of the length of the absorbent core.

Embodiments are based *inter alia* on the inventive insight that by providing an absorbent core comprising fluff pulp and substantially no superabsorbent polymers with one or more reinforcement zones, the wet integrity of the absorbent core is drastically improved. Especially by providing at least one reinforcement zone extending in the second and third zone of the absorbent core, it is achieved that the crotch region of the swim diaper exhibits an improved wet integrity thereby allowing the safe use of the swim diaper in a wet or aqueous environment. The crotch region of the swim diaper is the region of the diaper which has the smallest amount of support provided by the body of the wearer and is therefore the region of the diaper with the highest risk of sagging. By providing at least one reinforcement covering at least part of any area in the second and/or third zone having a width of 50% of a width of the absorbent core and a length of 40% of a length of the absorbent core, the amount of unsupported fluff pulp in the absorbent core is reduced, and a dry and/or wet integrity of the swim diaper is improved. In this manner, fecal excretions can be successfully retained within the swim diaper and contamination of the surroundings is avoided. Moreover, because the absorbent core is substantially free from superabsorbent polymers and because of the provision of one or more reinforcement zones the swim diaper will not, when saturated, become too heavy for the wearer and the risk of sagging is significantly reduced. This results in a swim diaper with an improved wearing comfort, both in and out of the water. Preferably the second zone and/or the third zone extend over a length corresponding with at least 20% of the length of the absorbent core, preferably at least 25% of the length of the absorbent core, more preferably at least 30% of the length of the absorbent core. In further embodiments the second zone and/or the third zone extend over an even larger length corresponding with at least 40% of the length of the absorbent core.

Preferably the one or more reinforcement zones are arranged such that for any area in the second and/or third zone having a width of 50% of the width of the absorbent core and a length of 35%, 30%, 25% or 20% of the length of the absorbent core, at least one reinforcement zone is at least partially located in the respective area.

Preferably the one or more reinforcement zones are arranged such that for any area in the second and/or third zone having a width of 40% of the width of the absorbent core and a length of 40%, 35%, 30%, 25% or 20% of the length of the absorbent core, at least one reinforcement zone is at least partially located in the respective area.

Preferably the one or more reinforcement zones are arranged such that for any area in the second and/or third zone having a width of 30% of the width of the absorbent core and a length of 40%, 35%, 30%, 25% or 20% of the length of the absorbent core, at least one reinforcement zone is at least partially located in the respective area.

The absorbent core comprises between 30 and 160 gsm of fluff pulp, preferably between 50 and 140 gsm of fluff pulp, and more preferably between 70 and 120 gsm of fluff pulp. It has been found by the Applicant that these grammages of fluff pulp, in combination with the one or more reinforcement zones in the absorbent core result in improved absorbent core functionality.

On the one hand, such grammages are sufficient to ensure that fecal excretions can be maintained within the swim diaper, and, on the other hand such grammages are beneficial in that they only allow for a limited amount of volume and/or weight increase of the swim diaper when absorbing liquid. In this manner a reliable and comfortable swim diaper is provided.

Preferably, the term "substantially free of superabsorbent polymer" implies that any superabsorbent polymer material represents less than 10% of the absorbent material in the absorbent core, preferably less than 5%, more preferably less than 3%, even more preferably less than 2%, most preferably less than 1%, and e.g. no superabsorbent polymer.

In an embodiment the one or more reinforcement zones extend from a crotch region in the direction of the front and/or rear transverse edge.

According to an embodiment the one or more reinforcement zones comprise a substantially continuous attachment having a width of at least 1 mm, preferably at least 2 mm, more preferably at least 3mm, and most preferably at least 4 mm. In that manner channels or pockets are created guiding the liquid to the absorbent areas. In addition, the one or more reinforcement zones may be configured to assist in creating a tub-shaped swim diaper, especially in the crotch region, which results in an improved fit of the swim diaper in wet conditions.

According to a further embodiment the one or more reinforcement zones comprise a discontinuous attachment at a plurality of locations at a distance of each other, the discontinuous attachment having a width of at least 1 mm, preferably at least 2 mm, more preferably at least 3mm, and most preferably at least 4 mm. Preferably the width direction corresponds with the transverse direction of the absorbent core.

Depending on the technique used for creating the one or more reinforcement zones either continuous or discontinuous attachments may be preferred. For example, when one or more adhesives are used to create the one or more reinforcement zones it is preferred that the reinforcement zones comprise substantially continuous attachments. In addition or alternatively, when sonic bonding is used to create to one or more reinforcement zones it is preferred that the reinforcement zones comprises a discontinuous attachment at a plurality of locations at a distance of each other.

When a discontinuous attachment is used, preferably, the discontinuous attachment comprises a plurality of attachment locations, seen in a width direction of the discontinuous attachment.

Preferably, there is substantially no absorbent material, more in particular substantially no fluff pulp, present in the one or more reinforcement zones. According to a preferred embodiment, outside of the one or more reinforcement zones the absorbent core has a maximum thickness; and the one or more reinforcement zones extend through at least 90 % of the maximum thickness of the absorbent core, more preferably through 95% of the thickness of the absorbent core, even more preferably through about 100% of the thickness of the absorbent core such that in the one or more reinforcement zones substantially no absorbent material is present between the top core wrap sheet and the back core wrap sheet.

According to an exemplary embodiment the first attachment zone and the second attachment zone are arranged symmetrically with respect to a longitudinal center line of the absorbent core extending between the first and second transverse edge.

Preferably, the one or more reinforcement zones comprise permanent attachments. Such permanent attachments will remain attached for at least 30 minutes after wetting, preferably for at least 60 minutes after wetting.

According to an embodiment the one or more reinforcement zones have a variable width. For example one reinforcement zone may have a varying width along its length, i.e. a smaller width in a back portion of the reinforcement zone and a larger width in a front portion of the reinforcement zone or vice versa. Alternatively or in addition, the one or more reinforcement zones may comprise a first reinforcement zone having a first width, and a second reinforcement zone having a second width, wherein the first width is different from the second width. In an embodiment there is provided a central reinforcement zone have a first width, and at least two reinforcement zones, one on either side of the central reinforcement zone, having a second width, wherein the second width is smaller than the first width.

In an exemplary embodiment the one or more reinforcement zones comprise at least a first elongate reinforcement zone extending in the second and/or third zone. The first elongate reinforcement zone may extend in a longitudinal or transverse direction, and more generally in any direction. Preferably, the first elongate reinforcement zone extends substantially in a longitudinal direction.

In a further embodiment the one or more reinforcement zones comprise at least a second elongate reinforcement zone extending in the second and/or third zone. The second elongate reinforcement zone may extend in a longitudinal or transverse direction, and more generally in any direction.

Preferably, the second elongate reinforcement zone extends substantially in a longitudinal direction.

Preferably the first and second elongate reinforcement zones extend next to each other. More preferably the first and second elongate reinforcement zones extend next to each other when looking in the longitudinal direction of the absorbent core.

In a preferred embodiment the first and second elongate reinforcement zone are arranged symmetrically with respect to a longitudinal center line of the absorbent core. In this manner, a good distribution of forces is achieved when the absorbent material, more in particular the fluff pulp, swells. Furthermore, this allows the swim diaper to adapt to the symmetry of the body of a wearer of the swim diaper.

Preferably a transverse distance between the first and second elongate reinforcement zone is between 10% and 50% of the width of the absorbent core, preferably between 15% and 45% of the width of the absorbent core, and more preferably between 20% and 40% of the width of the absorbent core. In this manner, seen in a width direction, a good distribution of forces is achieved in the absorbent core.

According to an embodiment the first elongate reinforcement zone extends from a front edge of the second zone in the direction of the third zone, and the second elongate reinforcement zone extends from a rear edge of the third zone in the direction of the second zone. In this manner, seen in a length direction, a good distribution of forces is achieved in the absorbent core.

In an exemplary embodiment the first elongate reinforcement zone is connected to the second elongate reinforcement zone through at least one connecting reinforcement zone.

According to an embodiment the at least one connecting reinforcement zone comprises at least one of a front connecting reinforcement zone which connects a front end portion of the first elongate reinforcement zone to a corresponding front end portion of the second elongate reinforcement zone; and a rear connecting reinforcement zone which connects a rear end portion of the first elongate reinforcement zone to a corresponding rear end portion of the second elongate reinforcement zone.

In a further embodiment the at least one connecting reinforcement zone extends substantially in a transverse direction of the absorbent core.

According to an embodiment the at least one connecting reinforcement zone comprise one or more straight portions, and/or one or more curved portions.

In a possible embodiment the first elongate reinforcement zone, the second elongate reinforcement zone, and/or the at least one connecting reinforcement zone collectively form a substantial "U" shape, or a substantial "V" shape. Preferably the at least one connecting reinforcement zone is a rear connecting reinforcement zone. In addition or alternatively, it is preferred that the first and second elongate reinforcement zones are diverging in the direction of the front edge of the absorbent core.

In an alternative embodiment the first elongate reinforcement zone, the second elongate reinforcement zone, and/or the at least one connecting reinforcement zone collectively form a substantially enclosed region.

In a further embodiment the substantially enclosed region has a substantial "O" shape, or a substantial polygon shape, such as a substantially rectangular shape, substantial triangular shape, a diamond shape, a substantially hexagonal shape.

According to an embodiment the first elongate reinforcement zone crosses a longitudinal center line of the absorbent core in at least a first crossing point; and said second elongate reinforcement zone crosses said longitudinal center line in at least a second crossing point. The first and second crossing point may be the same point or a different point, and may be located in the front part and/or in the rear part of the absorbent core. Preferably, the first and second elongate reinforcement zones collectively form a substantial "X" shape or a substantial "Y" shape.

In an embodiment the first zone extends over a length corresponding with at least 5%, preferably at least 10% of the length of the absorbent core seen in the longitudinal direction.

In a further embodiment the fourth zone extends over a length corresponding with at least 10% of the length of the absorbent core seen in the longitudinal direction, preferably at least 20%.

According to an embodiment the second and/or third zone each extend over a length corresponding with at least 25% of the length of the absorbent core seen in the longitudinal direction, preferably at least 30%.

In an exemplary embodiment at least one of the one or more reinforcement zones has a length which is larger than 5% of the length of the absorbent core, preferably larger than 10% of the length of the absorbent core, more preferably larger than 15% of the length of the absorbent core, most preferably larger than 20%, e.g. larger than 30% or even larger than 40%, 50%, 60%, 70%, 80% of the length of the absorbent core. More preferably, at least two of the one or more reinforcement zones have a length which is larger than 5% of the length of the absorbent core, preferably larger than 10% of the length of the absorbent core, more preferably larger than 15% of the length of the absorbent core, most preferably larger than 20%, e.g. larger than 30% or even larger than 40%, 50%, 60%, 70%, 80% of the length of the absorbent core. Most preferably, the first and second reinforcement zone extend next to each other, seen in a longitudinal direction, over a length larger than 10% of the length of the absorbent core, more preferably larger than 15% of the length of the absorbent core, most preferably larger than 20%, e.g. larger than 30% or even larger than 40%, 50%, 60%, 70%, 80% of the length of the absorbent core.

According to an embodiment the one or more reinforcement zones together cover at least 30%, preferably at least 40%, more preferably at least 50%, and most preferably at least 60% of the length of the absorbent core, e.g. at least 70% or even at least 80% of the length of the absorbent core.

In an embodiment wherein the attachment between the top core wrap sheet and the back core wrap sheet is achieved by any one of the following or a combination thereof: pressure bonding, thermal bonding, sonic bonding, chemical bonding, adhesive. If the attachment comprises an adhesive, preferably insoluble or water-resistant glue or adhesive is used.

In exemplary embodiments the one or more reinforcement zones comprise a third reinforcement zone.

In further embodiments the one or more reinforcement zones comprise a fourth reinforcement zone.

Preferably the third reinforcement zone and the fourth reinforcement zone are arranged symmetrically with respect to a longitudinal center line of the absorbent core. In an embodiment the first and second reinforcement zone are arranged in the front part of the absorbent core, and optionally extend into the rear part, and the third and fourth reinforcement zone are arranged in the rear part of the absorbent core, and optionally extend into the front part.

In an embodiment the one or more reinforcement zones extend at least partially in the first zone.

According to an embodiment the one or more reinforcement zones extend at least partially in the fourth zone.

In exemplary embodiments the one or more reinforcement zones comprise at least a transverse reinforcement zone extending in the transverse direction of the absorbent core, preferably over a length which is larger than 10%, more preferably larger than 15%, even more preferably larger than 20%, 25%, 30%, 35% or 40% of the width of the absorbent core.

Preferably, the transverse crotch line divides the absorbent core in the front part and the rear part on either side of the transverse crotch line. Preferably the distance between the transverse crotch line and a transverse center line passing through the middle of the absorbent core is less than 10% of the length of the absorbent core.

Preferably the swim diaper is a swim pant. In this manner, the swim pant can be applied to the wearer by first leading the feet into the respective leg openings and subsequently pulling the pants from the feet to waist area over the hips and buttocks of the wearer. The swim pant may include a front waist portion and a back waist portion which may be connected about the hips of the wearer by integral or releasable members. The swim pant may be preformed by any suitable technique including, but not limited to, joining together portions of the article using refastenable and/or nonrefastenable bonds (e.g., seam, weld, adhesive, cohesive bond, fastener, etc.). The swim pant may be preformed anywhere along the circumference of the article (e.g., side fastened, front waist fastened).

### BRIEF DESCRIPTION OF FIGURES

The accompanying drawings are used to illustrate presently preferred non-limiting exemplary embodiments of diapers according to the present invention. The above and other advantages of the features and objects of the invention will become more apparent and the invention will be better understood from the following detailed description when read in conjunction with the accompanying drawings, in which:
Figure 1A is a perspective view of an exemplary embodiment of a swim pant;
Figure 1B is a top plan view of the swim pant of figure 1A as a swim diaper;
Figure 1C is a schematic cross-section along the transverse center line TL of figure 1B;
Figure 2A is a top view of an exemplary embodiment of an absorbent core with four reinforcement zones using a first exemplary embodiment of a sealing pattern;
Figure 2B is a top view of an exemplary embodiment of an absorbent core with four reinforcement zones using a second exemplary embodiment of a sealing pattern;
Figure 2C is a top view of an exemplary embodiment of an absorbent core with four reinforcement zones using a third exemplary embodiment of a sealing pattern;
Figure 2D illustrates a fourth exemplary embodiment of a possible sealing pattern;
Figure 2E illustrates a fifth exemplary embodiment of a possible sealing pattern;
Figures 3A-3D illustrate exemplary embodiments of an absorbent core provided with one or more reinforcement zones for a swim diaper;
Figures 4A-4H illustrate exemplary embodiments of an absorbent core provided with one or more reinforcement zones for a swim diaper;
Figures 5A-5D illustrate exemplary embodiments of an absorbent core provided with one or more reinforcement zones for a swim diaper;
Figures 6A-6D illustrate exemplary embodiments of an absorbent core provided with one or more reinforcement zones for a swim diaper;
Figures 7A-7D illustrate exemplary embodiments of an absorbent core provided with one or more reinforcement zones for a swim diaper; and
Figures 8A-8D illustrate exemplary embodiments of an absorbent core provided with one or more reinforcement zones for a swim diaper.

### DESCRIPTION OF EMBODIMENTS

As used herein, the following terms have the following meanings:
"A", "an", and "the" as used herein refers to both singular and plural referents unless the context clearly dictates otherwise. By way of example, "an edge barrier" refers to one or more than one edge barrier.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or variations are appropriate to perform in the disclosed invention. However, it is to be understood that the value to which the modifier "about" refers is itself also specifically disclosed.

"Absorbent article", "absorbent garment", "absorbent product", "absorbing article", "absorbing garment", "absorbing product" and the like as used herein are used interchangeably and refer to devices that absorb and contain bodily exudates, and more specifically, refers to devices that are placed against or in proximity to the body of the wearer to absorb and contain the various liquids discharged from the body. Absorbent articles include but are not limited to feminine hygiene garments, baby diapers and pants, adult incontinence garments, various diaper and pants holders, liners, towels, absorbent inserts and the like.

"Absorbent core" as used herein refers to a three-dimensional part of the absorbent structure, comprising liquid-absorbing material, useful to permanently absorb and/or retain bodily exudates.

"Absorbent component" as used herein refers to a structural constituent of an absorbent article, e.g., a piece of an absorbent core, such as one of multiple pieces in a multi-piece absorbent core.

"Absorbent element" as used herein refers to a part of a functional constituent of an absorbent structure, e.g., a acquisition layer, a dispersion layer, core layer or a release structure formed of a material or materials having particular liquid handling characteristics suitable for the specific function.

"Absorbent fibrous polymer material" as used herein refers to an absorbent polymer material which is in threadlike from such as fibers, filaments, and the like so as to be less flowable in the dry state than particulates.

"Absorbent insert" as used herein refers to a device adapted for insertion into an "Absorbent layer" as used herein refers to a term referring to a discrete, identifiable sheet-like or web-like element of an absorbent article which may remain detached and relatively movable with respect to another such element or may be attached or joined so as to remain permanently associated with another such element. Each absorbent layer may itself include a laminate or combination of several layers, sheets and/or webs of similar or diverse compositions.

"Absorbent polymer material", "absorbent gelling material", "AGM", "superabsorbent", "superabsorbent material", "super absorbent polymer", "SAP" and the like as used herein are used interchangeably and refer to any suitable particulate (e.g., flaked, particulate, granular, or powdered) or fibrous cross linked polymeric materials that can absorb at least 5 times and

preferably at least about 10 times or more its weight of an aqueous 0.9% saline solution as measured using the Centrifuge Retention Capacity test (EDANA 441.2-01).

"Absorbent polymer material area" as used herein refers to the area of the absorbent structure wherein adjacent layers are separated by a multiplicity of absorbent polymer material. Incidental contact areas between these adjacent layers within the absorbent particulate polymer material area may be intentional (e.g bond area's) or unintentional (e.g. manufacturing artifacts).

"Absorbent particulate polymer material" as used herein refers to an absorbent polymer material which is in particulate form such as powders, granules, flakes and the like so as to be flowable in the dry state.

"Absorption" as used herein refers to the process by which a liquid is taken up within a material.

"Absorption rate" as used herein refers to the rate of absorption of liquid, i.e. the amount of liquid which is absorbed per unit of time, typically by an absorbent component, element and/or absorbent layer of the absorbent article, structure and/or core.

"Acquisition layer", "acquisition region", "acquisition surface" or "acquisition material" and the like as used herein refer to the layer overlying the absorbent core having a faster liquid uptake and/or distribution capability.

"Absorbency" is the ability of a material to take up fluids by various means including capillary, osmotic, solvent, chemical and/or other action.

"Adult incontinence garment" as used herein refers to absorbent articles intended to be worn by incontinent adults, for absorbing and containing bodily exudates.

"Adhesion" as used herein refers to the force that holds different materials together at their interface.

"Adhesive" as used herein refers to a material, which may or may not be flowable in solution or when heated, that is used to bond materials together.

"Adsorption" as used herein refers to the process by which a liquid is taken up by the surface of a material.

"Airlaying" as used herein refers to forming a web by dispersing fibers or particles in an air stream and condensing them from the air stream onto a moving screen by means of a pressure and/or vacuum; a web of fibers produced by airlaying is herein referred to an "airlaid"; an airlaid web bonded by one or more techniques to provide fabric integrity is herein referred to an "airlaid nonwoven".

"Apparent density", "density" as used herein refers to the basis weight of the sample divided by the caliper with appropriate unit conversions incorporated therein. Apparent density used herein has the unit g/cm³.

"Attach", "attached" and "attachment" as used herein are synonymous with their counterparts of the terms "fasten", "affix", "secure", "bind", "join" and "link".

"Baby diaper" as used herein refers to absorbent articles intended to be worn by children, for absorbing and containing bodily exudates which the user draws up between the legs and fastens about the waist of the wearer.

"Baby pants" as used herein refers to absorbent articles marketed for use in transitioning children from diapers to underwear intended to cover the lower torso of children, so as to absorb and contain body exudates which article is generally configured like a panty garment and manufactured with a completed waist encircling portion, thereby eliminating the need for the user to fasten the article about the waist of the wearer.

"Back region" as used herein refers to the portion of an absorbent article or part thereof that is intended to be positioned proximate the back of a wearer.

"Backing" as used herein refers to a web or other material that supports and reinforces the back of a product.

"Basis weight" is the weight per unit area of a sample reported in grams per square meter, g/m² or gsm.

"Bodily exudates", "body exudates", "bodily fluids", "body fluids", "bodily discharges", "body discharges", "fluid(s)", " liquid(s)", "fluid(s) and liquid(s) and the like as used herein are used interchangeably and refer to, but are not limited to urine, blood, vaginal discharges, breast milk, sweats and fecal matter.

"Binder", "adhesive", "glue", "resins", "plastics" and the like as used herein are used interchangeably and refer to substances, generally in a solid form (e.g. powder, film, fiber) or as a foam, or in a liquid form (e .g. emulsion, dispersion, solution) used for example by way of impregnation, spraying, printing, foam application and the like used for attaching or bonding functional and/or structural components, elements and materials, for example including heat and/or pressure sensitive adhesives, hot-melts, heat activated adhesives, thermoplastic materials, chemical activated adhesives/solvents, curable materials and the like.

"Bond strength" as used herein refers to the amount of adhesion between bonded surfaces. It is a measure of the stress required to separate a layer of material from the base to which it is bonded.

"Capillary action", "capillarity", or "capillary motion" and the like as used herein are used to refer to the phenomena of the flow of liquid through porous media.

"Chassis" as used herein refers to a foundational constituent of an absorbent article upon which the remainder of the structure of the article is built up or overlaid, e.g., in a diaper, the structural elements that give the diaper the form of briefs or pants when configured for wearing, such as a backsheet, a topsheet, or a combination of a topsheet and a backsheet.

"Cellulose fibers" as used herein refers to naturally occurring fibers based on cellulose, such as, for example cotton, linen, etc; wood pulp fibers are one example of cellulose fibers; man-made fibers derived from cellulose, such as regenerated cellulose (rayon), or partially or fully acetylated cellulose derivatives (e.g. cellulose acetate or triacetate) are also considered as cellulose fibers.

"Cluster" or the like as used herein refers to an agglomeration of particles and/or fibers.

"Chemically stiffened fibers", chemically modified fibers", "chemically cross-linked fibers", "curly fibers" and the like as used herein are used interchangeably and refer to any fibers which have been stiffened by chemical means to increase stiffness of the fibers under both dry and aqueous conditions, for example by way of addition of chemical stiffening agents (e.g. by coating, impregnating, etc), altering the chemical structure of the fibers themselves (e.g. by cross-linking polymer chains, etc) and the like.

"Cohesion" as used herein refers to the resistance of similar materials to be separated from each other.

"Compartment" as used herein refers to chambers, cavities, pockets and the like.

"Comprise," "comprising," and "comprises" and "comprised of" as used herein are synonymous with "include", "including", "includes" or "contain", "containing", "contains" and are inclusive or open-ended terms that specify the presence of what follows e.g. a component and do not exclude or preclude the presence of additional, non-recited components, features, elements, members, steps, known in the art or disclosed therein.

"Coverstock" as used herein refers to a lightweight non-woven material used to contain and conceal an underlying absorbent core material; examples are the facing layer or materials that cover the absorbent cores of feminine hygiene garment s, baby diapers and pants and adult incontinence garments.

"Crotch region" of an absorbent article as used herein refers to about 50% of the absorbent article's total length (i.e., in the y-dimension), where the crotch point is located in the longitudinal center of the crotch region. That is, the crotch region is determined by first locating the crotch point of the absorbent article, and then measuring forward and backward a distance of 25% of the absorbent article's total length.

"Cross direction (CD)", "lateral" or "transverse" and the like as used herein are used interchangeably and refer to a direction which is orthogonal to the longitudinal direction and includes directions within ±45° of the transversal direction.

"Curing" as used herein refers to a process by which resins, binders or plastics are set into or onto fabrics, usually by heating, to cause them to stay in place; the setting may occur by removing solvent or by cross-linking so as to make them in soluble.

"Diaper", "conventional diaper", "diaper-like", "diaper-like garment" and the like as used herein are used interchangeably and refer to disposable absorbent articles, which typically include a front waist portion and a back waist portion which may be releasable connected about the hips of the wearer during use by conventional fasteners such as adhesive tape fasteners or hook and loop type fasteners. In use, the article is positioned between the legs of the wearer and the fasteners are releasable attached to secure the back waist portion to the front waist portion of the diaper, thereby securing the diaper about the waist of the wearer. The front waist portion and a back waist portion are connected by relatively non-stretchable or stretchable members (the term "stretchable" as used herein refers to materials that are extensible when forces are applied to the material, and offer some resistance to extension). Hence, such articles are generally not configured to be pulled up or down over the hips of the wearer when the fasteners are attached.

"Dispersion layer", "dispersion region", "dispersion surface" or "dispersion material" and the like as used herein refer to the layer overlying the absorbent core having a faster liquid uptake and dispersion capability.

"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).

"Drylaying" as used herein refers to a process for making a nonwoven web from dry fiber; these terms apply to the formation of carded webs, as well as to the air laying formation of random webs; a web of fibers produced by drylaying is herein referred to as a "drylaid"; a drylaid web bonded by one or more techniques to provide fabric integrity is herein referred to a "drylaid nonwoven".

"Dry strength" as used herein refers to the strength of ajoint determined in dry state conditions, immediately after drying under specified conditions or after a period of conditioning in the standard laboratory atmosphere.

"Essentially cellulose free" or "little to no cellulose fibers" as used herein refers to an absorbent article, structure, core component and/or element containing less than 20% by weight cellulosic fibers, less than 10% cellulosic fibers, less than 5% cellulosic fibers, no cellulosic fibers, or no more than an immaterial amount of cellulosic fibers which do not materially affect the thinness, flexibility or absorbency thereof.

"Essentially fluffless" or "little to no fluff pulp" as used herein refers to an absorbent article, structure, core, component and/or element containing less than 20% by weight fluff pulp, less than 10% fluff pulp, less than 5% fluff pulp, no fluff pulp, or no more than an immaterial amount of fluff pulp which do not materially affect the thinness, flexibility or absorbency thereof.

"Fabric" as used herein refers to a sheet structure made from fibers, filaments and/or yarns.

"Feminine hygiene garments" as used herein refer to absorbent hygiene articles intended to be worn by woman, for absorbing and containing body exudates.

"Fiber" as used herein refers to the basic threadlike structure from which nonwovens, yarns and textiles are made. It differs from a particle by having a length at least 4 times its width; "Natural fibers" are either of animal (wool, silk), vegetable (cotton, flax, jute) or mineral (asbestos) origin, while "Man-made fibers" may be either polymers synthesized from chemical compounds (polyester, polypropylene, nylon, acrylic etc.) or modified natural polymers (rayon, acetate) or mineral (glass). "Fiber" and "filament" are used interchangeably.

"Fluff pulp" or "Pulp fluff" as used herein refers to wood pulp specially prepared to be drylaid. The fibers can be either natural or synthetic or a combination thereof.

"Front region" as used herein refers to the portion of an absorbent article or part thereof that is intended to be positioned proximate the front of a wearer.

"Garment facing layer" as used herein refers to elements of the chassis that form the outer surface of the absorbent article, such as the backsheet, the side panels, the waist fasteners, and the like, when such elements are present.

"Heat activated adhesive" as used herein refers to a dry adhesive that is rendered tacky or fluid by application of heat or heat and pressure to the assembly.

"Heat sealing adhesive" as used herein refers to a thermoplastic adhesive which is melted between the adherent surfaces by heat application to one or both of the adjacent adherent surfaces.

"High loft" as used herein refers to general term of low density, thick or bulky fabrics.

"Hot-melt adhesive" as used herein refers to a solid material that melts quickly upon heating, then sets to a firm bond upon cooling; used for almost instantaneous bonding.

"Hydrophilic" as used herein refers to having an affinity for being wetted by water or for absorbing water.

"Hydrophobic" as used herein refers to lacking the affinity for being wetted by water or for absorbing water.

"Immobilization layer" as used herein refers to a layer able to be applied to the absorbent polymer material or absorbent polymer material area with the intent to gather, bond and/or immobilize absorbent material and/or absorbent layer.

"Join", "joined" and "joining" as used herein refers to encompassing configurations wherein an element is directly secured to another element by affixing the element directly to the other element, as well as configurations wherein the element is indirectly secured to the other element by affixing the element to an intermediate member or members which in turn is or are affixed to the other element.

"Knitting" as used herein refers to the technique for interlocking loops of fibers with needles or similar devices.

"Layer" refers to identifiable components of the absorbent article, and any part referred to as a "layer" may actually comprise a laminate or combination of several sheets or webs of the requisite type of materials. As used herein, the term "layer" includes the terms "layers" and "layered." "Upper" refers to the layer of the absorbent article which is nearest to and/ or faces the wearer facing layer; conversely, the term "lower" refers to the layer of the absorbent article which is nearest to and/or faces the garment facing layer. "Layer" is three dimensional structure with a x dimension width, y dimension length, and z-dimensions thickness or caliper, said x-y dimensions being substantially in the plane of the article, however it should be noted that the various members, layers, and structures of absorbent articles according to the present invention may or may not be generally planar in nature, and may be shaped or profiled in any desired configuration .

"Machine direction (MD)", "longitudinal" and the like as used herein are used interchangeably and refer to a direction running parallel to the maximum linear dimension of the structure and includes directions within ±45° of the longitudinal direction.

"Major surface" as used herein refers to a term used to describe the surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.

"Mass flow" as used herein refers to the f low of a liquid f rom one absorbent element or component to another absorbent element or component by channel flow action.

"Mechanical bonding" as used herein refers to a method of bonding fibers by entangling them. This can be achieved by needling, stitching with fibers or by the use of high-pressure air or water jets and the like.

"Nonwoven" as used herein refers to manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes such as melt blowing, spun bonding, solvent spinning, electrospinning, and carding. The basis weight of nonwoven fabrics is usually expressed in grams per square meter (gsm).

"Pant", "training pant", "closed diapers", "prefastened diapers", "pull-on diapers" and "diaper-pants" and the like as used herein are used interchangeably and refer to absorbent articles which are typically applied to the wearer by first leading the feet into the respective leg openings and subsequently pulling the pants from the feet to waist area over the hips and buttocks of the wearer and which are capable of being pulled up or down over the hips of the wearer. Typically, such articles may include a front waist portion and a back waist portion which may be connected about the hips of the wearer by integral or releasable members. A pant may be preformed by any suitable technique including, but not limited to, joining together portions of the article using refastenable and/or nonrefastenable bonds (e.g., seam, weld, adhesive, cohesive bond, fastener, etc.). A pant may be preformed anywhere along the circumference of the article (e.g., side fastened, front waist fastened).

"Polymer" as used herein refers to but is not limited to, homopolymers, copolymers, such as for example, block, graft, random and alternating copolymers, terpolymers, etc. and blends and modifications thereof. Unless otherwise specifically limited, the term "polymer" includes all possible spatial configurations of the molecule and include, but are not limited to isotactic, syndiotactic and random symmetries.

"Rear" as used herein refers to the portion of an absorbent article or part thereof that is intended to be positioned proximate the back of the wearer.

"Release structure", "release region", "release surface" or "release material" and the like as used herein are used interchangeably and refer to a structure in fluid communication with the absorbent core having a larger relative liquid absorption capacity and/or rate allowing it to quickly take up, temporarily hold and releasing liquids.

"Resin" as used herein refers to a solid or semisolid polymeric material.

"Thermobonding" as used herein refers to a method of bonding fibers by the use of heat and/or high-pressure.

"Thermoplastic" as used herein refers to polymeric materials that have a melting temperature and can flow or be formed into desired shapes on the application of heat at or below the melting point.

"Ultrasonic" as used herein refers to the use of high frequency sound to generate localized heat through vibration thereby causing thermoplastic fibers to bond to one another.

"Water-absorbing", "liquid-absorbing", "absorbent", "absorbing" and the like as used herein are used interchangeably and refer to compounds, materials, products that absorb at least water, but typically also other aqueous fluids and typically other parts of bodily exudates such as at least urine or blood.

"Wearer facing layer" as used herein refers to elements of the chassis that form the inner surface of the absorbent article, such as the topsheet, the leg cuffs, and the side panels, etc., when such elements are present.

"Weaving" as used herein refers to the process of interlacing two or more sets of yarns at right angles to form a fabric; a web of fibers produced by weaving is herein referred to as a "woven".

"Web material" as used herein refers to an essentially endless material in one direction, i.e. the longitudinal extension or the length, or the x- direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Typically, the width of web materials (they-direction) will be significantly larger than the thickness, but less than the length. Often, though not necessarily, the thickness and the width of such materials is essentially constant along the length of the web. Without intending any limitation, such web materials may be cellulosic fiber materials, tissues, woven or nonwoven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, nonwoven/film laminates. Web materials may comprise other materials, such as added binding material, particles, hydrophilizing agents and the like.

"Wet burst strength" is a measure of a layer's ability to absorb energy, when wet and subjected to deformation normal to the plane of the web.

"Wet strength" as used herein refers to the strength of a joint determined immediately after removal from a liquid in which it has been immersed under specified conditions of time, temperature and pressure. The term is commonly used in the art to designate strength after immersion in water.

"Wetlaying" as used herein refers to the forming a web from an aqueous dispersion of fibers by applying modified paper making techniques; a web of fibers produced by wetlaying is herein referred to as a "wetlaid".

"Wood pulp" as used herein refers to cellulosic fibers used to make viscose rayon, paper and the absorbent cores of products such as feminine hygiene garments, baby diapers and pants and adult incontinence garments.

"X-y dimension" as used herein refers to the plane orthogonal to the thickness of the article, structure or element. The x- and y-dimensions correspond generally to the width and length, respectively, of the article, structure or element.

"Z-dimension" as used herein refers to the dimension orthogonal to the length and width of the article, structure or element. The z-dimension corresponds generally to the thickness of the article, structure or element.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

The same or similar features and components are indicated with the same reference numerals throughout the figures.

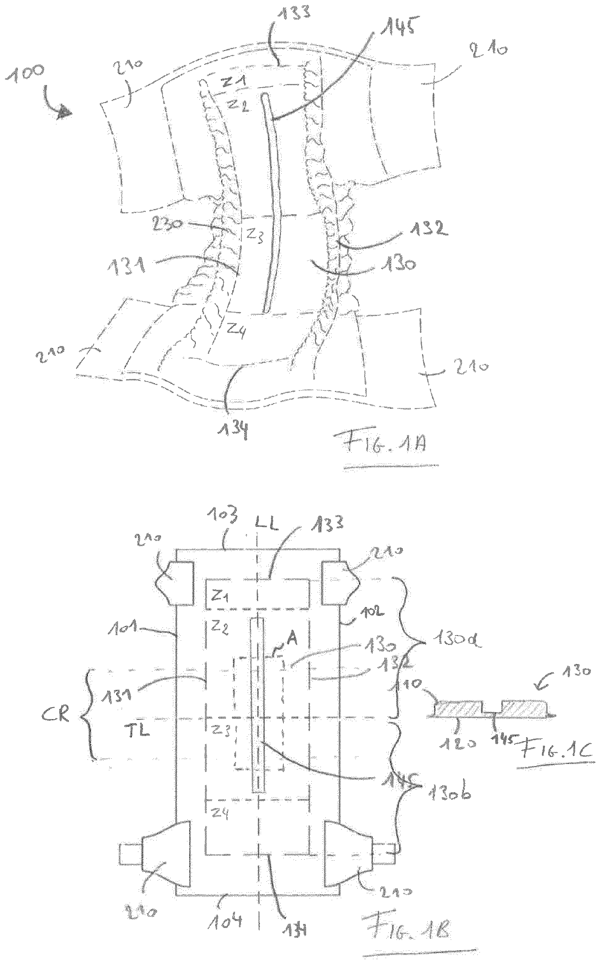

Figures 1A and 1B illustrate corresponding exemplary embodiments of a swim pant and a swim diaper, respectively. Figure 1B shows the swim diaper in its flat out, un-contracted state with the wearer side facing the viewer. The skilled person understands that the swim diaper may also be a pant, as illustrated in figure 1A, which is considered as being a specific type of swim diaper. It is clear to the skilled person that any embodiment described in view of swim diapers, is applicable in a similar manner to swim pants, mutatis mutandis. The swim diaper 100 comprises a liquid pervious topsheet, a liquid impervious backsheet, and an absorbent core 130 positioned in between the topsheet and the backsheet. The absorbent core 130 comprises fluff pulp between a top core wrap sheet 110 and a back core wrap sheet 120 and is substantially free of superabsorbent polymers. Absorbent core 130 has a first and second longitudinal edge 131, 132 and a front and rear transverse edge 133, 134.

The absorbent core 130 is provided with a reinforcement zone 145. In the reinforcement zone the top core wrap sheet 110 is attached to the back core wrap sheet 120. It is clear for the skilled person that more than one reinforcement zone 145 may be present in the absorbent core 130, as will further be elaborated in view of further embodiments. The illustrated reinforcement zone 145 extends from a crotch region CR in the direction of the front and rear transverse edge 133, 134. In the one or more reinforcement zones 145 the top core wrap sheet 110 is attached to the back core wrap sheet 120 along an attachment which extends, seen in a transverse direction of the absorbent core, over a transverse distance which is at least 1 mm, preferably at least 2 mm, more preferably at least 3mm, most preferably at least 4mm. In addition, or alternatively, the top core wrap sheet 110 is attached to the back core wrap sheet 120 along a discontinuous attachment at a plurality of locations at a distance of each other, seen in the transverse direction of the absorbent core.

As illustrated in figure 1C the reinforcement zone 145 has a bottom where the top core wrap sheet 110 is attached to back core wrap sheet 120. The width w of the bottom, seen in a transverse direction of absorbent core 130, is preferably larger than 2 mm, more preferably larger than 3 mm and even more preferable larger than 4 mm. To that end the attachment between top core wrap sheet 110 and the back core wrap sheet 120 may be an attachment extending over a transverse distance which is at least 2 mm, preferably at least 3 mm, more preferably at least 4 mm; and/or the attachment may be a discontinuous attachment in a plurality of locations at a distance of each other, seen in a transverse direction of absorbent core 130. Preferably the attachment at the bottom between the top core wrap sheet and the back core wrap sheet is realized by any one of the following or a combination thereof: pressure bonding, thermobonding, sonic bonding, chemical bonding, adhesive, mechanical bonding.

It is illustrated in figure 1C that the top core wrap sheet 110 is attached to the back core wrap sheet 120 at a bottom such that that a lower reinforcement zone 145 is formed. However, in alternative embodiments the actual attachment may be positioned at different locations, e.g. the attachment zone may be positioned more or less centrally such that a middle reinforcement zone is created, or the attachment may be positioned at the top such that an upper reinforcement zone is created.

It is clear to the skilled person that the reinforcement zones may be provided by means of continuous attachments in the transversal direction of the absorbent core and/or continuous attachments in the longitudinal direction of the absorbent core and/or discontinuous attachments in the transversal direction of the absorbent core and/or discontinuous attachments in the longitudinal direction of the absorbent core.

Absorbent core 130 has a front part 130a extending at one side of a transverse crotch line which corresponds in this embodiment with line TL, and a rear part 130b extending at the other side of the transverse crotch line TL. Seen in a longitudinal direction of the absorbent core 130, looking from the front transverse edge 133 to the rear transverse edge 134, the absorbent core 130 comprises subsequently a first Z1, second Z2, third Z3 and fourth zone Z4. The first and second zone Z1, Z2 extend in the front part 130a of the absorbent core 130 and said third and fourth zone Z3, Z4 extend in the rear part 130b. It is noted that although in figure 1B the rear edge of the second zone Z2 coincides with the transverse center line TL, the second zone Z2 may extend beyond the transverse center line TL. In a similar manner, it is noted that although in figure 1B the front edge of the third zone Z3 coincides with the transverse center line TL, the third zone Z3 may extend beyond the transverse center line TL. In the illustrated embodiment the first zone Z1 at the front edge of the absorbent core is the smallest zone and extends e.g. over 5-15% of the length of the absorbent core. The fourth zone Z4 at the rear edge of the absorbent core is the second smallest zone and extends over e.g. 15-25% of the length of the absorbent core. The second zone Z2 is the biggest zone and extends e.g. over about 35-45% of the length of the absorbent core. The third zone Z3 is the second biggest zone and extends e.g. over about 25-35% of the length of the absorbent core. It is noted that the lengths of the first, second, third and fourth zone Z1, Z2, Z3 and Z4 may be different in other embodiments. Preferably, the second zone and third zone together extend over a length corresponding with at least 40% of the length of the absorbent core, preferably at least 50% of the length of the absorbent core. Preferably the second zone and/or the third zone extend over a length corresponding with at least 20% of the length of the absorbent core, preferably at least 25% of the length of the absorbent core, more preferably at least 30% of the length of the absorbent core. In further embodiments the second zone and/or the third zone extend over an even larger length corresponding with at least 40% of the length of the absorbent core. Preferably the first zone Z1 extends over a length corresponding with at least 5%, more preferably at least 10% of the length of the absorbent core seen in the longitudinal direction. Preferably the fourth zone Z4 extends over a length corresponding with at least 10% of the length of the absorbent core seen in the longitudinal direction, more preferably at least 20%. Preferably the second and third zone Z2, Z3 each extend over a length corresponding with at least 25% of the length of the absorbent core seen in the longitudinal direction, more preferably at least 30%.

The one or more reinforcement zones 145 may be indicated with a color and/or with a pattern which is different from the color and/or pattern of the topsheet of the swim diaper or pant. More in particular the area the reinforcement zones may comprise a print allowing a user to visually distinguish one or more of the reinforcement zones. This print may be arranged on the topsheet, on the top core wrap sheet, on the back core wrap sheet, on the backsheet, or on any sheet in between the topsheet and the backsheet, as long as it is visible for a user. As the sheets may be partially transparent, the print may be arranged on a sheet in between the topsheet and the backsheet, as long as it is visible through the topsheet and/or the backsheet. Preferably the print is visible when looking at the topsheet of the diaper. In that manner a user will be able to easily recognize the front and rear portion of a swim diaper, and will recognize more easily how to put on the swim diaper.

The chassis of the swim diaper or swim pant 100 in figures 1A-1D comprises a liquid pervious topsheet (not shown in figures 1C and 1D, but the topsheet is a layer above top core wrap sheet 110) and liquid impervious backsheet (not shown in figures 1C and 1D, but the backsheet is a layer below back core wrap sheet 110). The topsheet may be attached to the top core wrap sheet 110, e.g. in the one or more reinforcement zone 145. Also, the backsheet may be attached to the back core wrap sheet 120, e.g. in the one or more reinforcement zone 145. Preferably the chassis further includes side panels or ears 210, elasticized leg cuffs 230 and elastic waist elements (not shown). A front end portion of swim diaper 100 is configured as a front waist region. The opposite rear end portion is configured as a back waist region of swim diaper 100. An intermediate portion of swim diaper 100 is configured as crotch region CR, which extends longitudinally between first and second waist regions. The first and second waist regions may include elastic waist elements such that they gather about the waist of the wearer to provide improved fit and containment. Crotch region CR is that portion of diaper 100 which, when the diaper 100 is worn, is generally positioned between the wearer's legs. The periphery of diaper 100 is defined by the outer edges of the diaper 100 in which longitudinal edges 101, 102 run generally parallel to a longitudinal axis of diaper 100 and transverse end edges 103, 104 run between the longitudinal edges 101, 102 generally parallel to a transverse axis of diaper 100. In the case of a swim diaper the chassis also comprises a fastening system, which may include at least one fastening or securing member and at least one landing zone. In the case of a swim pant no additional fastening system is provided. The various components within swim diaper 100 may be bound, joined or secured by any method known in the art, for example by adhesives in uniform continuous layers, patterned layers or arrays of separate lines, spirals or spots. Top core wrap sheet, topsheet, back core wrap sheet, backsheet, absorbent material and other components may be assembled in a variety of well-known configurations and are well known in the art.

The backsheet covers absorbent core 130 and preferably extends beyond the absorbent core 130 toward longitudinal edges 101, 102 and transverse edges 103, 104 of swim diaper 100 and may be joined with the top sheet. The backsheet prevents bodily exudates absorbed by the absorbent core 130 and contained within diaper 100 from soiling the environment. In preferred embodiments, the backsheet is substantially impervious to bodily exudates and comprises a laminate of a nonwoven and a thin plastic film such as a thermoplastic film. The backsheet may comprise breathable materials that permit vapor to escape from diaper 100 while still preventing bodily exudates from passing through backsheet. It may be semi-rigid, non-elastic and can be made fully or partially elasticized and include backing.

The topsheet which is located above the top core wrap sheet 110, is preferably soft, exhibits good strikethroughs and has a reduced tendency to rewet from the liquid absorbent material. The topsheet may be semi-rigid and non-elastic, or may be fully or partially elasticized. The topsheet is intended to be placed in close proximity to the skin of the wearer when swim diaper 100 is worn. The topsheet permits bodily exudates to rapidly penetrate it so as to flow more quickly toward absorbent core 130 via a top surface thereof, preferably not allowing such bodily exudates to flow back through the topsheet. The topsheet may be constructed from any one of a wide range of liquid and vapor permeable, preferably hydrophilic, materials. The upper and lower surface of topsheet may be treated differently. The topsheet may include e.g. a surfactant on the upper surface so as to facilitate liquid transfer there through, especially at a central zone or area of the topsheet located over absorbent core 130, and/or a hydrophobic agent on the lower surface to minimize the liquid contained within absorbent core 130 from contact wetting topsheet thereby reducing rewet values. The topsheet may be coated with a substance having rash preventing or rash reducing properties. Preferably, topsheet covers substantially the entire wearer facing area of diaper 100, including substantially all of the front waist region, back waist region, and crotch region CR. Optionally, side panels 210, 210' and/or waist feature layers of the inner region may be formed from the same single topsheet material. Alternatively, the topsheet may be formed from multiple different materials which vary across the topsheet. Such a multiple piece design allows for creation of preferred properties in different zones of the topsheet.

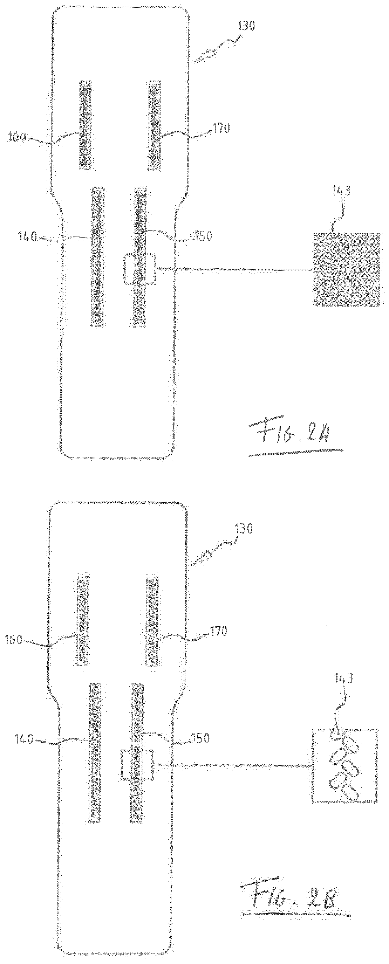

Absorbent core 130 comprises fluff material, typically cellulosic fluff pulp, and is substantially free from superabsorbent polymers. Absorbent core 130 may be configured to extend over substantially the full length and/or width of swim diaper 100. However, as in the embodiment of figures 1A-1C, preferably absorbent structure 130 is not coextensive with the entire swim diaper 100 and is limited to certain regions of swim diaper 100 including crotch region CR. In various embodiments, the absorbent core 130 extends to the edges of swim diaper 100 but the fluff material is concentrated in the crotch region CR or another target zone of the diaper 100. In figures 1A-1C, absorbent core 130 is shown as having a substantially rectangular configuration, however, absorbent core 130 may be shaped differently, such as, elliptical, dogbane shaped, T-shaped or I-shaped. More in particular the width of the front portion may be larger than the width of the rear portion of the absorbent core 130, or vice versa. Reference is for example made to the embodiments of figures 2A-2C, wherein the front portion of the absorbent core is wider than the rear portion. For embodiments where the absorbent core has different widths along its length, the wording "width of the absorbent core" preferably refers to a maximum width of the absorbent core. However, in other embodiments with a variable width, it may also refer to the average width of the absorbent core along its length.

Swim diaper 100 may also utilize a pair of containment walls or cuffs 230. Each cuff 230 is a longitudinally extending wall structure preferably positioned on each side of absorbent core 130 and spaced laterally from the transverse center line TL. Preferably, cuffs 230 are attached, for example, by adhesive or sonic bonding to the lower structure. Preferably, cuffs 230 are equipped with elastic members. When released or otherwise allowed relaxing, the elastic members retract inwardly. When swim diaper 100 is worn, the elastic members function to contract cuffs 230 about the buttocks and the thighs of the wearer in a manner, which forms a seal between swim diaper 100, the buttocks and the thighs.

The front and rear waist regions each comprise a central region and a pair of side panels or ears 210 which typically comprise the outer lateral portions of the waist regions. These side panels 210 may be unitary with the chassis or may be attached or joined thereto by any means know in the art. Preferably, the side panels 210 positioned in the back waist region are flexible, extensible and/or elastic in at least the lateral direction. In another embodiment the side panels 210 are non-elastic, semi-rigid, rigid and/or stiff. In order to keep swim diaper 100 in place about the wearer, preferably at least a portion of the back waist region is attached by fastening or securing members to at least a portion of the front waist region. The fastening or securing members may be e.g. adhesive, mechanical fasteners, hook and loop features, conceivable strings and/or combinations thereof. The fastening or securing members may also be co-adhesive such that they adhere to each other but not other materials. Preferably the materials making up the fastening or securing members are flexible, extensible and/or elastic, allowing them to better conform to the shape and movements of the body and thus, to reduce the likelihood that the fastening system will irritate or injure the wearer's skin. Alternatively, the swim diaper may be configured as a pant or the like. In this configuration, the swim diaper may or may not have fastening members.

Swim diaper 100 may also employ additional layers, such as an acquisition layer and/or dispersion layer situated between topsheet and absorbent core 130, and/or coverstock layers, and/or other layers situated between absorbent core 130 and backsheet. An acquisition layer and/or dispersion layer serves to slow down the flow so that the liquid has adequate time to be absorbed by absorbent core 130.

Swim diaper 100 may also include such other features, components and elements as are known in the art including waistbands, waist cap features, elastics and the like to provide better fit, containment and aesthetic characteristics. These features may be assembled in a variety of well-known configurations and are well known in the art.

The elongated reinforcement zone 145 of swim diaper 100 extends from the crotch region CR in the direction of both the front and rear transverse edge 133, 134. The reinforcement zone 145 extends over substantially the entire length of the second zone Z2 and third zone Z3, along a longitudinal center line LL. In this manner it is guaranteed that a maximum free transverse distance, i.e. a transverse distance along which the absorbent core does not comprise a reinforcement zone, in the second zone Z2 and third zone Z3 is smaller than 50% of the width of the absorbent core. In other words, the reinforcement zone is arranged such that for any area in the second and third zone having a width of 50% of a width of the absorbent core and a length of 40% of a length of the absorbent core, the reinforcement zone is at least partially located in the respective area. In the event of a variable with of the absorbent core, the width may correspond with the maximum width. To further illustrate this, an area A having the above described dimensions (the drawing is not to scale) is schematically drawn in figure 1B, for example in a position such that the area A partially overlaps the second and third zone. For any position of the area A within the second and/or third zone, the reinforcement zone 145 will be at least partially located within the area A. In other embodiments, more than one reinforcement zone may be at least partially present within the area A with the above described dimensions. Consequently, according to further embodiments the one or more reinforcement zones are arranged such that for any area A in the second and/or third zone having a width of 50%, 45%, 40%, 35% or 30% of the width of the absorbent core and a length of 40%, 35%, 30%, 25% or 20% of the length of the absorbent core, at least one reinforcement zone is at least partially located in the respective area A. In other words, the dimensions of the area A are representative for a "density" of reinforcement zones, i.e. if the dimensions of the area A correspond with the smaller percentages of the abovementioned ranges, this is indicative of a relatively high density of reinforcement zones in the second and/or third zone. On the other hand, if the dimensions of the area A correspond with the larger percentages of the abovementioned ranges, this is indicative of a relatively low density of reinforcement zones in the second and/or third zone. A higher density of reinforcement zones may result in an even higher wet integrity of the absorbent core as compared to a lower density of reinforcement zones. However, it should be noted that the density of reinforcement zones cannot be endlessly increased as this might deteriorate the absorbent and/or containment functionality of the swim diaper.

The reinforcement zone 145 comprises a substantially continuous attachment having a width of at least 1 mm, preferably at least 2 mm, more preferably at least 3mm, and most preferably at least 4 mm. Alternatively, or in addition, the one or more reinforcement zone may comprises a discontinuous attachment at a plurality of locations at a distance of each other, the discontinuous attachment having a width of at least 1 mm, preferably at least 2 mm, more preferably at least 3mm, and most preferably at least 4 mm, wherein the width is defined as a total width of the attachments at the plurality of locations. Reference is made to figures 2A-2E.

Figure 2A illustrates an exemplary embodiment of an absorbent core 130 with four reinforcement zones 140, 150, 160, 170. In the embodiment of figure 11A, the attachments at the reinforcement zones are formed by welding the top core wrap sheet 110 to the back core wrap sheet 120. This welding may be done according to a predetermined sealing pattern. In the embodiment of figure 11A, the pattern consists of a plurality of discrete shapes 143, here a plurality of squares. Preferably, the discrete shapes 143 have dimensions smaller than 2 mm. Preferably, the distance between adjacent discrete shapes is between 0.5 and 3 mm.

Figure 2B illustrates another exemplary embodiment of a sealing pattern that may be used to create reinforcement zones. Here the pattern consists of a plurality of discrete shapes in the form of rounded elements 143. The rounded elements may have a length dimension between 0.5 mm and 5 mm, and a width dimension between 0.5 mm and 5 mm. Preferably, the discrete shapes are equally distributed in the reinforcement zones.

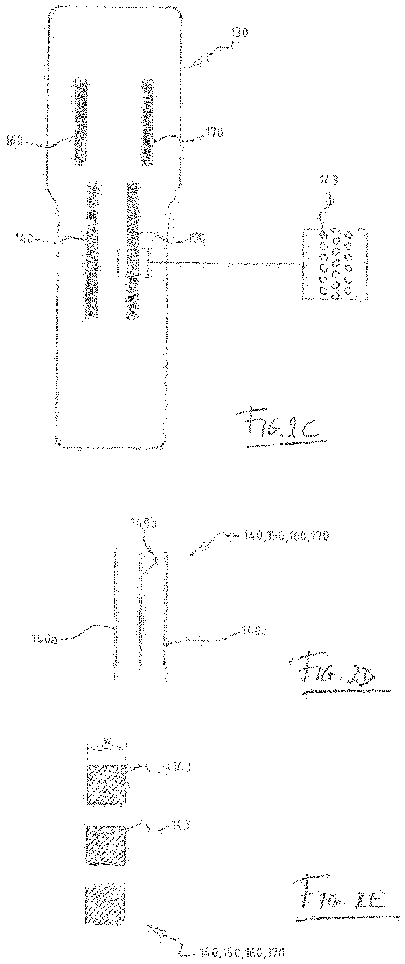

Figure 2C illustrates yet another embodiment where the sealing pattern consists of discrete shapes which are rounded. In this embodiment, three columns of rounded discrete elements 143 are used for each reinforcement zone 140, 150, 160, 170.

Figure 2D illustrates another exemplary embodiment of attachments of reinforcement zones 140, 150, 160, 170. In this embodiment, the reinforcement zones are formed by a plurality of continuous line-shaped attachments 140a, 140b, 140c. The number of lines used may vary, and may be e.g. two lines or more than three adjacent lines. Preferably, the distance w between a first line 140a and a last line 140c is at least 1 mm, more preferably at least 2 mm, even more preferably more than 4 mm.

In the exemplary embodiment of figure 2E, the attachments of the reinforcement zones 140, 150, 160, 170 may be formed of a plurality of discrete elements 143, wherein each discrete element has a width w which covers the entire width w of the reinforcement zone.

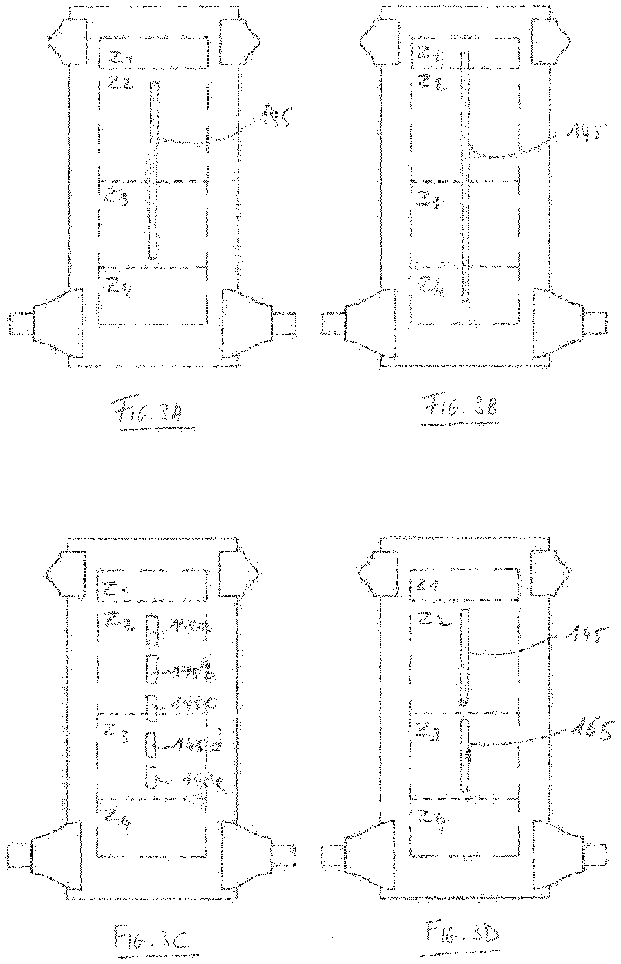

Figures 3A-3D illustrate multiple advantageous positions for the one or more reinforcement zones in the absorbent core of a swim diaper. In the illustrated embodiments the one or more reinforcement zones are substantially aligned and positioned along the longitudinal center line of the absorbent core.

In figure 3A the elongate reinforcement zone 145 extends between a front edge of the second zone Z2 and a rear edge of the third zone Z3. The reinforcement zone 145 is orientated along the longitudinal center line LL of the absorbent core and has a width, in the transverse direction, of about 4-5 mm. The reinforcement zone 145 does not extend over the entire length of the second and third zone Z2, Z3. However, the distance between a front end of the reinforcement zone 145 and the front edge of the second zone Z2, and the distance between a rear end of the reinforcement zone 145 and the rear edge of the third zone Z3 is smaller than 20% of the length of the absorbent core. In this manner, it is guaranteed that in the second zone Z2 and third zone Z3 no area having a width of 50% of the width of the absorbent core and having a length of 40%, preferably 30%, more preferably 20% of the length of the absorbent core can be appointed which is free of reinforcement zone(s).

It is noted that although in most of the figures the rear edge of the second zone Z2 coincides with the transverse center line TL, the second zone Z2 may extend beyond the transverse center line TL. In a similar manner, it is noted that although in the figures the front edge of the third zone Z3 coincides with the transverse center line TL, the third zone Z3 may extend beyond the transverse center line TL. In most of the illustrated embodiments, the first zone Z1 at the front edge of the absorbent core is the smallest zone and extends e.g. over about 10% of the length of the absorbent core. The fourth zone Z4 at the rear edge of the absorbent core is the second smallest zone and extends e.g. over about 20% of the length of the absorbent core. The second zone Z2 is the biggest zone and extends e.g. over about 40% of the length of the absorbent core. The third zone Z3 is the second biggest zone and extends e.g. over about 30% of the length of the absorbent core. It is noted that the lengths of the first, second, third and fourth zone Z1, Z2, Z3 and Z4 may be different in other embodiments. Preferably, the second zone and third zone together extend over a length corresponding with at least 40% of the length of the absorbent core, preferably at least 50% of the length of the absorbent core. Preferably the second zone and/or the third zone extend over a length corresponding with at least 20% of the length of the absorbent core, preferably at least 25% of the length of the absorbent core, more preferably at least 30% of the length of the absorbent core. In further embodiments the second zone and/or the third zone extend over an even larger length corresponding with at least 40% of the length of the absorbent core. Preferably the first zone Z1 extends over a length corresponding with at least 5%, more preferably at least 10% of the length of the absorbent core seen in the longitudinal direction. Preferably the fourth zone Z4 extends over a length corresponding with at least 10% of the length of the absorbent core seen in the longitudinal direction, more preferably at least 20%. Preferably the second and third zone Z2, Z3 each extend over a length corresponding with at least 25% of the length of the absorbent core seen in the longitudinal direction, more preferably at least 30%.

In figure 3B the reinforcement zone 145 extends beyond the second and third zone Z2, Z3. The front end of the reinforcement zone 145 is situated in the first zone Z1, and the rear end of the reinforcement zone 145 is situated in the fourth zone Z4. In this manner, additional reinforcement is provided to the absorbent core. More in particular, the reinforcement zone 145 is arranged such that for any area in of the absorbent core having a width of 50% of the width of the absorbent core and a length of 10% of the length of the absorbent core, a reinforcement zone is at least partially located in said area.

In figure 3C a plurality of reinforcement zones 145a, 145b, 145c, 145d, 145e is provided in the absorbent core. Each of the reinforcement zones has a length of about 10% of the length of the absorbent core, and they are spaced apart by a distance of about 5% of the length of the absorbent core, thereby forming a discontinuous attachment along the longitudinal center line LL of the absorbent core.

In figure 3D two reinforcement zones 145, 165 are provided. In the illustrated embodiment reinforcement zone 145 is located in the second zone Z2 and reinforcement zone 165 is located in the third zone Z3.

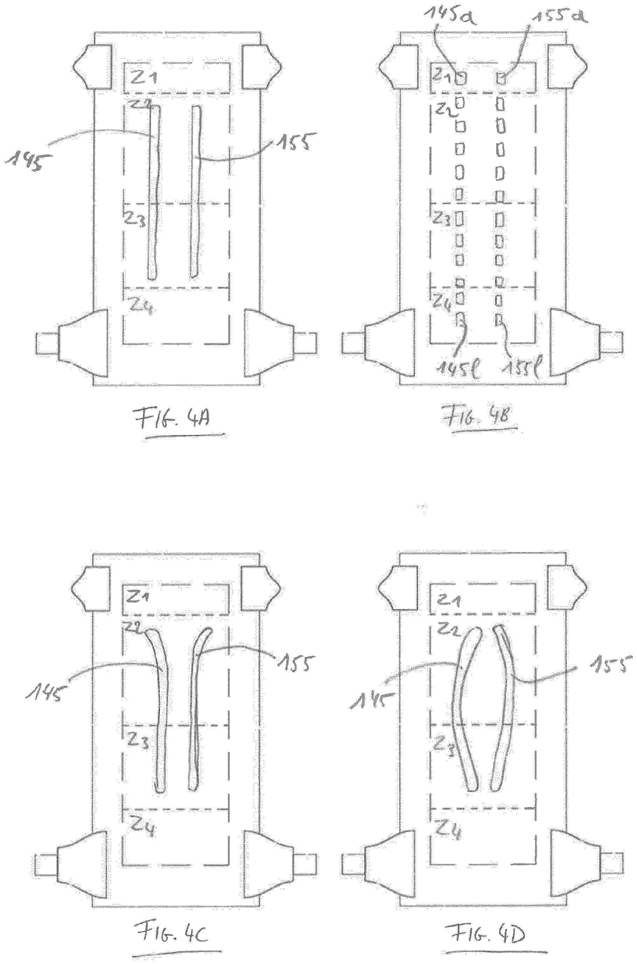

Figures 4A-4G illustrate multiple advantageous embodiments wherein at least two reinforcement zones 145, 155 are provided which extend next to each other, and wherein the reinforcement zones 145, 155 are arranged symmetrically with respect to the longitudinal center line LL of the absorbent core.

In figure 4A a first reinforcement zone 145 and a second reinforcement zone 155 are provided, which extend substantially in the longitudinal direction of the absorbent core and which are arranged symmetrically with respect to a longitudinal center line of the absorbent core. The first reinforcement zone 145 does not extend over the entire length of the second and third zone Z2, Z3. However, the distance between a front end of the first reinforcement zone 145 and the front edge of the second zone Z2, and the distance between a rear end of the first reinforcement zone 145 and the rear edge of the third zone Z3 is smaller than 20% of the length of the absorbent core. In other embodiments the first reinforcement zone 145 may be at least partially located in the first zone Z1 and/or fourth zone Z4. The second reinforcement zone 155 does not extend over the entire length of the second and third zone Z2, Z3. However, the distance between a front end of the second reinforcement zone 155 and the front edge of the second zone Z2, and the distance between a rear end of the reinforcement zone 145 and the rear edge of the third zone Z3 is smaller than 20% of the length of the absorbent core. In other embodiments the second reinforcement zone 155 may be at least partially located in the first zone Z1 and/or fourth zone Z4. In the illustrated configuration the transverse distance between the first and second reinforcement zone 145, 155 is substantially the same along the length of the reinforcements zones 145, 155 and corresponds with about 35% of the width of the absorbent core. In this manner, it is guaranteed that in the second zone Z2 and third zone Z3 no area having a width of 35% of the width of the absorbent core and having a length of 40%, preferably 30%, more preferably 20% of the length of the absorbent core can be appointed which is free of reinforcement zone(s). In other embodiments, the transverse distance between the first and second reinforcement zone may be anywhere between 10% and 50% of the width of the absorbent core, preferably between 15% and 45% of the width of the absorbent core, and more preferably between 20% and 40% of the width of the absorbent core.

In figure 4B the first reinforcement zone comprises a plurality of smaller attached reinforcement zones 145a-145l, and the second reinforcement zone comprises a plurality of smaller attached reinforcement zone 155a-155l. Each of the smaller reinforcement zones has a length of about 5% of the length of the absorbent core, and they are spaced apart by a distance of about 5% of the length of the absorbent core, thereby forming two discontinuous elongate reinforcement zones, one on each side of the longitudinal center line LL of the absorbent core. The smaller reinforcement zones 145a and 155a are located in the first zone Z1, whereas the smaller reinforcement zones 1451 and 1551 are located in the fourth zone Z4. In this manner, additional reinforcement is provided to the absorbent core outside of the second and third zone Z2, Z3.

Figure 4C illustrates a similar embodiment as shown in figure 4A. However, the reinforcement zones 145, 155 in figure 4A are substantially straight, whereas the reinforcement zones 145, 155 in figure 4C comprise at least one curved portion. More in particular, the front end of the first reinforcement zone 145 bends towards the first longitudinal edge 131 of the absorbent core and the front end of the second reinforcement zone 155 bends towards the second longitudinal edge 132 of the absorbent core. Consequently a transverse distance between the first and second reinforcement zones 145, 155 in the rear part of the absorbent core is smaller than a transverse distance between the first and second reinforcement zones 145, 155 in the front part of the absorbent core.

Figure 4D illustrates a similar embodiment as shown in figure 4A. However, the reinforcement zones 145, 155 in figure 4A are substantially straight, whereas the reinforcement zones 145, 155 in figure 4D comprise at least one curved portion. More in particular, the first and second reinforcement zones 145, 155 are curved to approach each other at their respective front ends and rear ends. Consequently a transverse distance between the first and second reinforcement zones 145, 155 at the front and rear ends thereof is smaller than a transverse distance between the first and second reinforcement zones 145, 155 in the middle parts thereof.

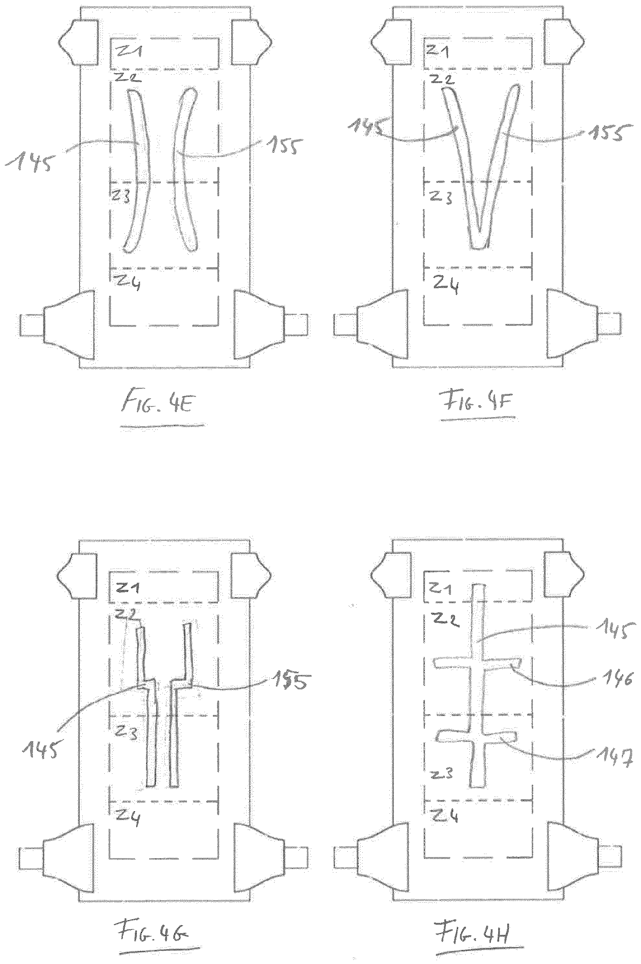

Figure 4E illustrates a similar embodiment as shown in figure 4A. However, the reinforcement zones 145, 155 in figure 4A are substantially straight, whereas the reinforcement zones 145, 155 in figure 4E comprise at least one curved portion. More in particular, the first and second reinforcement zones 145, 155 are curved to bend away from each other at their respective front ends and rear ends. Consequently a transverse distance between the first and second reinforcement zones 145, 155 at the front and rear ends thereof is larger than a transverse distance between the first and second reinforcement zones 145, 155 in the middle parts thereof. For example, the distance between the middle parts of the first and second reinforcement zones 145, 155 may be at least 5% of the width of the absorbent core, in figure 4E about 30%, and the distance between the front and rear ends of the first and second reinforcement zones 145, 155 may be larger, in figure 4E about 60% of the width of the absorbent core. The front ends of the first and second reinforcement zones 145, 155 are located in the second zone Z2. However, in other embodiments these front ends may be located in the first zone Z1. The rear ends of the first and second reinforcement zones 145, 155 are located in the third zone Z3. However, in other embodiments these rear ends may be located in the fourth zone Z4.

Figure 4F illustrates an embodiment wherein the first and second reinforcement zones 145, 155 are angled with respect to the longitudinal center line of the absorbent core. In addition, the first reinforcement zone 145 is connected to the second reinforcement zone 155 such that the reinforcement zones 145, 155 together form a substantially V-shaped attachment.

In figure 4G the on ore more reinforcement zones comprise a first reinforcement zone 145 and a second reinforcement zone 155. The first reinforcement zone 145 comprises a longitudinal front portion and a longitudinal rear portion connected to each other by means of a transverse connecting portion. The second reinforcement zone 155 comprises a longitudinal front portion and a longitudinal rear portion connected to each other by means of a transverse connecting portion. The longitudinal front portions extend from the crotch region in the direction of the front transverse edge, and are interconnected via transverse connecting portions to the longitudinal rear portions extending from the crotch region to the rear transverse edge, respectively. In figure 4G the distance between the longitudinal rear portions is smaller than the distance between the longitudinal front portions of the reinforcement zones 145, 155. However, in other embodiment the distance between the longitudinal rear portions may be larger than the distance between the longitudinal front portions of the reinforcement zones 145, 155.

In figure 4H one elongated reinforcement zone 145 is provided extending substantially longitudinally from the first zone Z1 to the third zone Z3. In addition two substantially transverse reinforcement zones 146, 147 are provided at a distance from each other. Each transverse reinforcement zone 146, 147 crosses the longitudinal reinforcement zone 145. Transverse reinforcement zone 146 crosses the longitudinal reinforcement zone 145 in the second zone Z2. Transverse reinforcement zone 147 crosses the longitudinal reinforcement zone 145 in the third zone Z3.

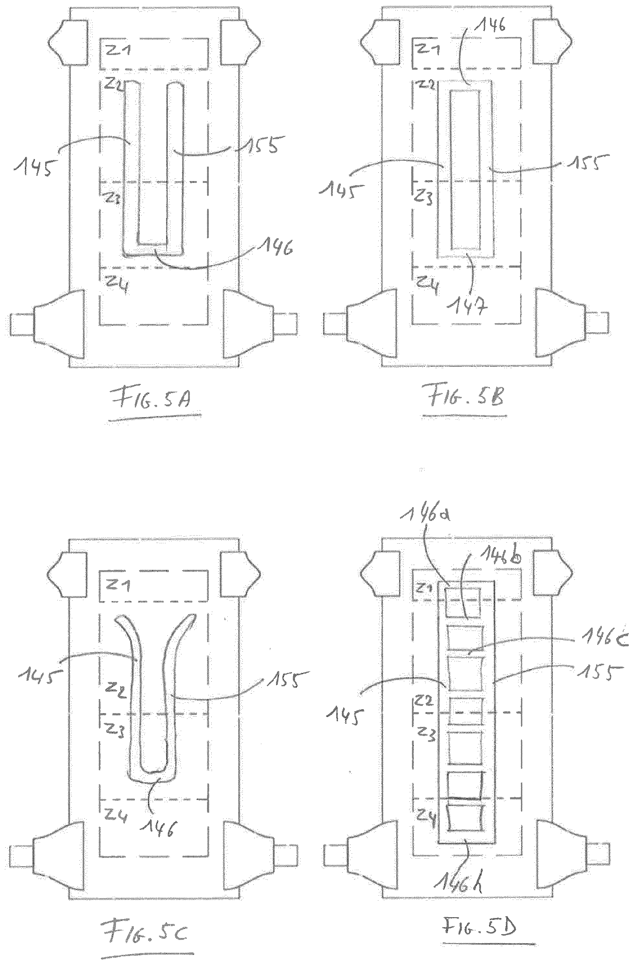

Figures 5A-5D illustrate multiple advantageous embodiments wherein at least two reinforcement zones 145, 155 are provided which extend next to each other, and wherein at least one connecting reinforcement zone 146, 147 is provided which connects the two reinforcements zones 145, 155.

The embodiment of figure 5A corresponds with the embodiment of figure 4A, with the difference that in figure 5A a connecting reinforcement zone 146 is provided. In this example the connecting reinforcement zone 146 is substantially straight and extends substantially transversely between the rear ends of the first and second elongated reinforcement zones 145, 155, such that the reinforcement zones 145, 155 and 146 together form a substantially U-shaped attachment.

Alternatively, or in addition, the connecting reinforcement zone may be provided between the front ends of the first and second reinforcement zones 145, 155, and/or between any other portions of the first and second reinforcement zone 145, 155, respectively. It is clear to the skilled person that one or more connecting reinforcement zone may be provided which is straight, curved and/or angled.

Figure 5B illustrates an embodiment which is similar to the embodiment of figure 5A with the addition of a connecting reinforcement zone between the front ends of the first and second elongated reinforcement zones 145, 155 such that a total of two connecting reinforcement zones 146, 147 are provided. The reinforcement zones 145, 155, 146, 147 together form an enclosed region in the shape of a rectangle.

The embodiment of figure 5C corresponds with the embodiment of figure 4C, with the difference that in figure 5C a connecting reinforcement zone 146 is provided. In this example the connecting reinforcement zone 146 extends substantially transversely between the rear ends of the first and second elongated reinforcement zones 145, 155, such that the reinforcement zones 145, 155 and 146 together form a substantially U-shaped attachment. Alternatively, or in addition, the connecting reinforcement zone may be provided between the front ends of the first and second reinforcement zones 145, 155, and/or between any other portions of the first and second reinforcement zone 145, 155, respectively. It is clear to the skilled person that one or more connecting reinforcement zone may be provided which is straight, curved and/or angled.

In figure 5D the first and second elongate reinforcement zones 145, 155 extend from the first zone Z1 to the fourth zone Z4 of the absorbent core. A plurality of connecting transverse reinforcement zones 146a-146h is provided. Connecting reinforcement zone 146a provides a connection between the front ends of the elongate reinforcement zones 145, 155, connecting reinforcement zone 146h provides a connection between the rear ends of the elongate reinforcement zones 145, 155, and connecting reinforcement zones 146b-146g provide further connections between the elongate reinforcement zones 145, 155.

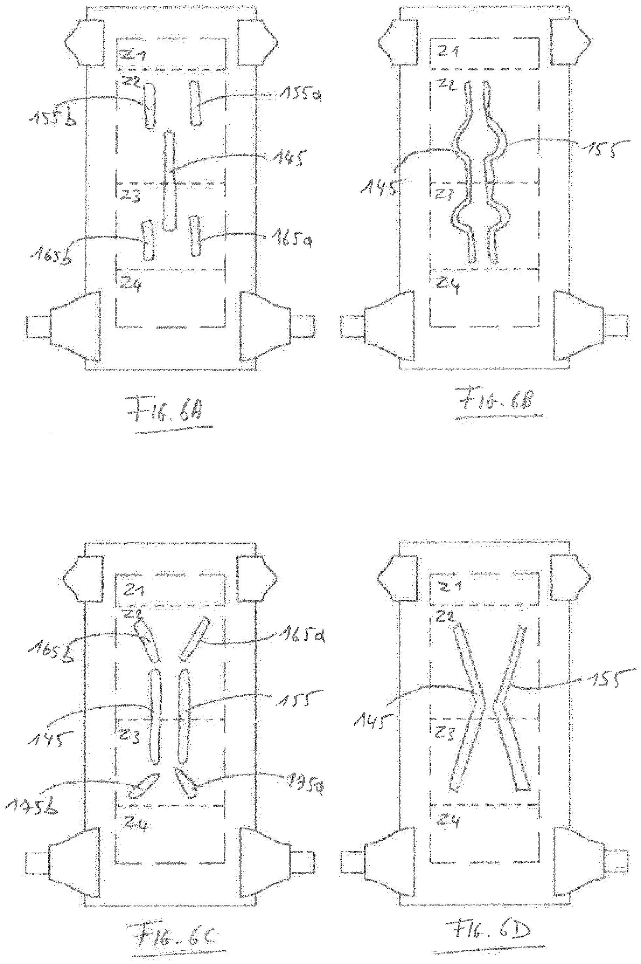

In figure 6A the absorbent core of the swim diaper is provided with a central reinforcement zone 145, which is surrounded by one or more front reinforcement zones 155a, 155b and/or one or more rear reinforcement zones 165a, 165b. The central reinforcement zone extends in the second zone Z2 and third zone Z3 of the absorbent core. The front reinforcement zones 155a, 155b extend in the second zone Z2 of the absorbent core. The rear reinforcement zones 165a, 165b extend in the third zone Z3 of the absorbent core.

In the embodiment of figure 6B the one or more reinforcement zones comprise a first undulated reinforcement zone 145 and a second undulated reinforcement zone 155 each extending over at least 60% of the length of the absorbent core. The undulations increase the length of the reinforcements further improving the dry and wet integrity of the absorbent core.

In figure 6C the absorbent core of the swim diaper is provided with two central reinforcement zones 145, 155. In addition one or more front reinforcement zones 165a, 165b and/or one or more rear reinforcement zones 175a, 175b are provided. The central reinforcement zones extend in the second zone Z2 and third zone Z3 of the absorbent core. The front reinforcement zones 165a, 165b extend in the second zone Z2 of the absorbent core and are angles with respect to the longitudinal direction of the absorbent core. The rear reinforcement zones 175a, 175b extend in the third zone Z3 of the absorbent core and are angled with respect to the longitudinal direction of the absorbent core.

Figure 6D illustrates that the first and second reinforcement zones 145, 155 may comprise various rectilinear sections which are oriented at an angle with respect to the longitudinal direction of the absorbent core. More in particular, the first and second reinforcement zones 145, 155 are angled as to move away from each other at their respective front ends and rear ends. Consequently a transverse distance between the first and second reinforcement zones 145, 155 at the front and rear ends thereof is larger than a transverse distance between the first and second reinforcement zones 145, 155 in the middle parts thereof. For example, the distance between the middle parts of the first and second reinforcement zones 145, 155 may be at least 5% of the width of the absorbent core, and the distance between the front and rear ends of the first and second reinforcement zones 145, 155 may be larger, in figure 6D about 65% of the width of the absorbent core. The front ends of the first and second reinforcement zones 145, 155 are located in the second zone Z2. However, in other embodiments these front ends may be located in the first zone Z1. The rear ends of the first and second reinforcement zones 145, 155 are located in the third zone Z3. However, in other embodiments these rear ends may be located in the fourth zone Z4.

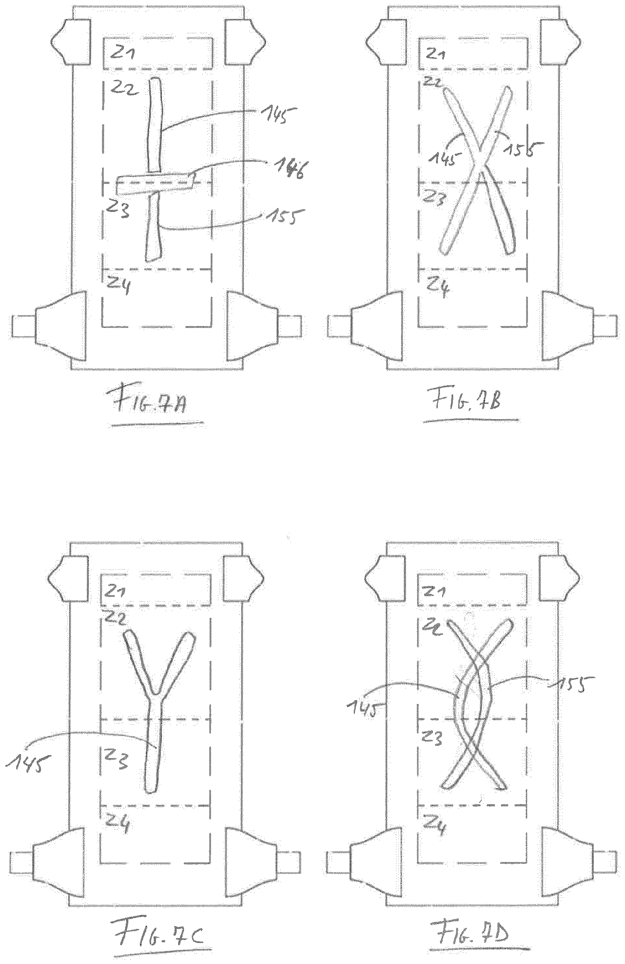

In figure 7A a front elongate reinforcement zone 145 and a rear elongate reinforcement zone 155 are provided. The front reinforcement zone 145 extends in the second zone Z2. The rear reinforcement zone 155 extends in the third zone Z3. The front end of the rear reinforcement zone 155 is located at a distance of the rear end of the front reinforcement zone 145. Between the front and rear reinforcement zones 145, 155 a transverse reinforcement zone 146 is provided. The front, rear and transverse reinforcement zones 145, 155, 146 form a substantially cross-shaped attachment.

Figure 7B illustrates that the first and second reinforcement zones 145, 155 may comprise various rectilinear sections which are oriented at an angle with respect to the longitudinal direction of the absorbent core. More in particular, the first and second reinforcement zones 145, 155 cross each other to form a substantially X-shaped attachment.

Figure 7C illustrates that the first reinforcement zone 145 may comprise various rectilinear sections which are oriented at an angle with respect to the longitudinal direction of the absorbent core. More in particular, the first reinforcement zone 145 comprises one substantially longitudinal portion in the third zone Z3 of the absorbent core, and two angles portions in the second zone Z2 of the absorbent core to form a substantially Y-shaped attachment.

According to the exemplary embodiment of figure 7D the one or more reinforcement zone comprises a first reinforcement zone 145 and a second reinforcement zone 155. The first and second reinforcement zones 145, 155 extend from the crotch region in the direction of the front and rear transverse edge of the absorbent core, and are curved such that the first and second reinforcement zones 145, 155 cross each other at a first crossing point in the front part of the absorbent core and in a second crossing point in the rear part of the absorbent core.

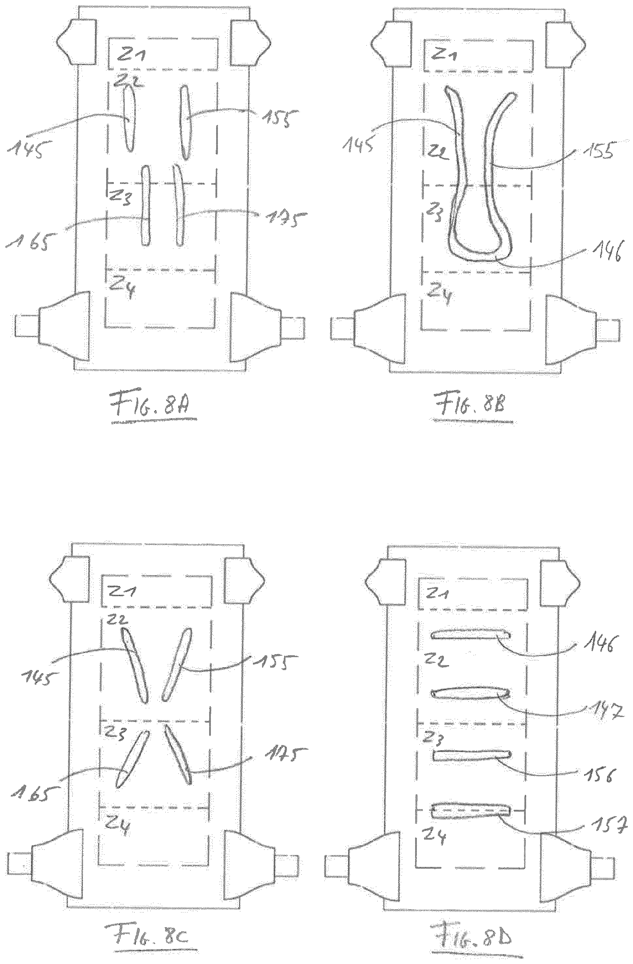

In figure 8A the absorbent core is provided with a plurality of reinforcement zones 145, 155, 165, 175 comprising at least a first reinforcement zone 145 and a second reinforcement zone 155. The first and second attachment zones extend next to each other from the crotch region CR in the direction of the front transverse edge. In the first and second reinforcement zone 145, 155 the top core wrap sheet is attached to the back core wrap sheet. The first and second reinforcement zone 145, 155 are located a distance of each other. Preferably the distance d12 is between 10 mm and 50 mm, more preferably between 15 and 30 mm. Preferably, the length of the first and second reinforcement zone 145, 155 is substantially the same, more preferably the length of the first reinforcement zone and the length of the second reinforcement zone is between 60 mm and 140 mm, more preferably between 75 mm and 125 mm. Preferably, the distance between the first reinforcement zone 145 and the first longitudinal edge 131 is between 20 and 30 mm, and the distance between the second reinforcement zone 155 and the second longitudinal edge 132 is between 20 and 30 mm. First reinforcement zone 145 and second reinforcement zone155 are substantially parallel and run in the longitudinal direction of absorbent core. However, it is also possible for first and second reinforcement zone145, 155 to extend under an angle with respect to the longitudinal direction of absorbent core, e.g. an angle between 5 and 45°. For example, first and second reinforcement zone 145, 155 may be diverging slightly outwardly in the direction of the front edge. The absorbent core is further provided with a third and a fourth reinforcement zone 165, 175 located at a distance of each other. Third and fourth reinforcement zone 165, 175 each extend from crotch region CR in the direction of the rear edge. The distance between first and second reinforcement zone 145, 155 is different from the distance between third and fourth reinforcement zone 165, 175. Preferably the distance is between 25 mm and 80 mm, more preferably between 35 mm and 55 mm. Preferably, the length of the third and fourth reinforcement zone 165, 175 is substantially the same, more preferably the length of the third reinforcement zone and the length 14 of the fourth reinforcement zone is between 30 mm and 130 mm, more preferably between 30 mm and 70 mm. Preferably, the distance between the third reinforcement zone 165 and the first longitudinal edge 131 is between 20 and 30 mm, and the distance between the fourth reinforcement zone 175 and the second longitudinal edge 132 is between 20 and 30 mm. Third reinforcement zone 165 and fourth reinforcement zone 175 are substantially parallel and run in the longitudinal direction of absorbent core. However, it is also possible for the third and fourth reinforcement zone 165, 175 to extend under an angle with respect to the longitudinal direction of absorbent core, e.g. an angle between 5 and 45°. For example, third and fourth reinforcement zone 165, 175 may be diverging slightly outwardly in the direction of rear transverse edge. Preferably third reinforcement zone 165 and fourth reinforcement zone 175 are arranged symmetrically with respect to the longitudinal center line LL of the absorbent core.

The embodiment of figure 8B corresponds with the embodiment of figure 4E, with the difference that in figure 8B a connecting reinforcement zone 146 is provided. In this example the connecting reinforcement zone 146 is curved and extends substantially transversely between the rear ends of the first and second elongated reinforcement zones 145, 155, such that the reinforcement zones 145, 155 and 146 together form a substantially U-shaped attachment. Alternatively, or in addition, the connecting reinforcement zone may be provided between the front ends of the first and second reinforcement zones 145, 155, and/or between any other portions of the first and second reinforcement zone 145, 155, respectively. It is clear to the skilled person that one or more connecting reinforcement zone may be provided which is straight, curved and/or angled.

In figure 8C the absorbent core is provided with four reinforcement zones 145, 155, 165, 175. Two front reinforcement zones 145, 155 extend in the second zone Z2 under an angle with the longitudinal direction of the absorbent core. Two rear reinforcement zones 165, 175 extend in the third zone Z3 under an angle with the longitudinal direction of the absorbent core. The front reinforcement zones 145, 155 and rear reinforcement zones 165, 175 together form a substantially X-shaped attachment.

In the embodiment of figure 8D a plurality of substantially transverse reinforcement zones 146, 147, 156, 157 is provided in the absorbent core. Reinforcement zones 146 and 147 extend in the second zone Z2 of the absorbent core, whereas reinforcement zones 156 and 157 extend in the third zone Z3 of the absorbent core. In the illustrated example reinforcement zone 157 partially extends in the fourth zone Z4 of the absorbent core. A longitudinal distance between adjacent reinforcement zones is smaller than 20% of the length of the absorbent core, preferably smaller than 15% of the length of the absorbent core, and more preferably smaller than 10% of the length of the absorbent core.

Whilst the principles of the invention have been set out above in connection with specific embodiments, it is to be understood that this description is merely made by way of example and not as a limitation of the scope of protection which is determined by the appended claims.

## Claims

1. A swim diaper (100) comprising a liquid pervious topsheet, a liquid impervious backsheet, and an absorbent core (130) positioned in between said topsheet and said backsheet, said absorbent core having a first and second longitudinal edge (131, 132) and a front and rear transverse edge (133, 134);
wherein the absorbent core (130) comprises a top core wrap sheet (110) and a back core wrap sheet (120) and comprises between 30 and 160 gsm fluff pulp in between the top and back core wrap sheet; wherein the absorbent core is substantially free of superabsorbent polymers;
wherein, seen in a longitudinal direction of the absorbent core, looking from the front transverse edge to the rear transverse edge, the absorbent core comprises subsequently a first (Z1), second (Z2), third (Z3) and fourth zone (Z4);
wherein the absorbent core (130) comprises a front part (130a) extending between the front edge (133) and a transverse crotch line of the absorbent core (130), and a rear part (130b) extending between the rear edge (134) and the transverse crotch line of the absorbent core (130); wherein said first (Z1) and second zone (Z2) extend in the front part (130a) of the absorbent core (130) and said third (Z3) and fourth zone (Z4) extend in the rear part (130b), wherein the second (Z2) and third zone (Z3) together extend over a length corresponding with at least 40% of the length of the absorbent core (130); and
wherein the absorbent core (130) is provided with one or more reinforcement zones (145), where the top core wrap sheet (110) is attached to the back core wrap sheet (120), extending at least partially in the second (Z2) and/or third zone (Z3);
wherein the one or more reinforcement zones (145) are arranged such that for any area in the second (Z2) and/or third zone (Z3) having a width of 50% of a width of the absorbent core (130) and a length of 40% of a length of the absorbent core (130), at least one reinforcement zone (145) is at least partially located in said area.

2. Swim diaper (100) according to claim 1, wherein the second (Z2) and third zone (Z3) together extend over a length corresponding with at least 50% of the length of the absorbent core (130).

3. Swim diaper (100) according to claim 1 or 2, wherein each one of the second (Z2) and third zone (Z3) extends over a length corresponding with at least 20% of the length of the absorbent core (130), preferably at least 25% of the length of the absorbent core (130).

4. Swim diaper (100) according to any one of the preceding claims, wherein the absorbent core (130) comprises between 50 and 140 gsm of fluff pulp, and preferably between 70 and 120 gsm of fluff pulp.

5. Swim diaper (100) according to any one of the preceding claims, wherein the one or more reinforcement zones (145) extend from a crotch region in the direction of the front and/or rear transverse edge (133, 134).

6. Swim diaper (100) according to any one of the preceding claims, wherein the one or more reinforcement zones (145) comprise a substantially continuous attachment having a width of at least 1 mm, preferably at least 2 mm, more preferably at least 3mm, and most preferably at least 4 mm.

7. Swim diaper (100) according to any one of the preceding claims, wherein the one or more reinforcement zones (145) comprise a discontinuous attachment at a plurality of locations at a distance of each other, the discontinuous attachment having a width of at least 1 mm, preferably at least 2 mm, more preferably at least 3mm, and most preferably at least 4 mm.

8. Swim diaper (100) according to any one of the preceding claims, wherein the one or more reinforcement zones (145) comprise at least a first elongate reinforcement zone extending in the second (Z2) and/or third zone (Z3).

9. Swim diaper (100) according to any one of the preceding claims, wherein, the one or more reinforcement zones (145) are one or more permanent attachment zones, said permanent attachment zones being configured to remain attached upon wetting, preferably for at least 30 minutes.

10. Swim diaper (100) according to the previous claim, wherein the one or more reinforcement zones (145) comprise at least a second elongate reinforcement zone extending in the second (Z2) and/or third zone (Z3).

11. Swim diaper (100) according to the previous claim, wherein the first and second elongate reinforcement zones extend next to each other.

12. Swim diaper (100) according to claim 10 or 11, wherein the first and second elongate reinforcement zone are arranged symmetrically with respect to a longitudinal center line of the absorbent core (130).

13. Swim diaper (100) according to any one of claims 10 - 12, wherein a transverse distance between the first and second elongate reinforcement zone is between 10% and 50% of the width of the absorbent core (130), preferably between 15% and 45% of the width of the absorbent core (130), and more preferably between 20% and 40% of the width of the absorbent core (130); and/or
wherein the first elongate reinforcement zone extends from a front edge of the second zone (Z2) in the direction of the third zone (Z3), and wherein the second elongate reinforcement zone extends from a rear edge of the third zone (Z3) in the direction of the second zone.

14. Swim diaper (100) according to any one of claims 10 - 13, wherein the first elongate reinforcement zone is connected to the second elongate reinforcement zone through at least one connecting reinforcement zone; and/or
wherein the at least one connecting reinforcement zone comprises at least one of:
- a front connecting reinforcement zone which connects a front end portion of the first elongate reinforcement zone to a corresponding front end portion of the second elongate reinforcement zone;
- a rear connecting reinforcement zone which connects a rear end portion of the first elongate reinforcement zone to a corresponding rear end portion of the second elongate reinforcement zone; and/or
wherein the at least one connecting reinforcement zone extends substantially in a transverse direction of the absorbent core (130); and/or
wherein the at least one connecting reinforcement zone comprise one or more straight portions, and/or one or more curved portions; and/or
wherein the first elongate reinforcement zone, the second elongate reinforcement zone, and/or the at least one connecting reinforcement zone collectively form a substantial "U" shape, or a substantial "V" shape; and/or
wherein the first elongate reinforcement zone, the second elongate reinforcement zone, and the at least one connecting reinforcement zone collectively form a substantially enclosed region; and/or
wherein the substantially enclosed region has a substantial "O" shape, or a substantial polygon shape, such as a substantially rectangular shape, substantial triangular shape, a diamond shape, a substantially hexagonal shape; and/or
wherein said first elongate reinforcement zone crosses a longitudinal center line of the absorbent core (130) in at least a first crossing point; and said second elongate reinforcement zone crosses said longitudinal center line in at least a second crossing point; wherein said first and second crossing point are the same point or a different point, and are located in the front part and/or in the rear part of the absorbent core (130).

15. Swim diaper (100) according to any one of the preceding claims, wherein the first zone (Z1) extends over a length corresponding with at least 5%, preferably at least 10% of the length of the absorbent core (130) seen in the longitudinal direction; and/or
wherein the fourth zone (Z4) extends over a length corresponding with at least 10% of the length of the absorbent core (130) seen in the longitudinal direction, preferably at least 20%; and/or
wherein the second (Z2) and/or third zone (Z3) each extend over a length corresponding with at least 25% of the length of the absorbent core (130) seen in the longitudinal direction, preferably at least 30%; and/or
wherein each one of the one or more reinforcement zones (145) has a length which is larger than 5% of the length of the absorbent core (130), preferably larger than 10% of the length of the absorbent core (130), more preferably larger than 15% of the length of the absorbent core (130); and/or
wherein the one or more reinforcement zones (145) together cover at least 30%, preferably at least 40%, more preferably at least 50%, and most preferably at least 60% of the length of the absorbent core (130); and/or
wherein the attachment between the top core wrap sheet (110) and the back core wrap sheet (120) is any one of the following or a combination thereof: pressure bonding, thermal bonding, sonic bonding, chemical bonding, adhesive; and/or
wherein the one or more reinforcement zones (145) comprise a third reinforcement zone; wherein preferably the one or more reinforcement zones (145) comprise a fourth reinforcement zone; wherein preferably the third reinforcement zone and the fourth reinforcement zone are arranged symmetrically with respect to a longitudinal center line of the absorbent core (130); and/or
wherein the one or more reinforcement zones (145) extend at least partially in the first zone; and/or
wherein the one or more reinforcement zones (145) extend at least partially in the fourth zone; and/or
wherein the one or more reinforcement zones (145) comprise at least a transverse reinforcement zone extending in the transverse direction of the absorbent core (130); and/or
wherein the swim diaper (100) is a swim pant.

## Patentansprüche

1. Schwimmwindel (100), umfassend eine flüssigkeitsdurchlässige Oberschicht, eine flüssigkeitsundurchlässige Unterschicht und einen zwischen der Oberschicht und der Unterschicht angeordneten Absorptionskern (130), wobei der Absorptionskern einen ersten und einen zweiten Längsrand (131, 132) und einen vorderen und einen hinteren Querrand (133, 134) aufweist;
wobei der Absorptionskern (130) eine obere Kernhüllschicht (110) und eine untere Kernhüllschicht (120) umfasst und zwischen 30 und 160 g/m² Flockenzellstoff zwischen der oberen und unteren Kernhüllschicht umfasst; wobei der Absorptionskern im Wesentlichen frei von superabsorbierenden Polymeren ist;
wobei der Absorptionskern, in Längsrichtung des Absorptionskerns gesehen, vom vorderen Querrand zum hinteren Querrand blickend, nacheinander eine erste (Z1), zweite (Z2), dritte (Z3) und vierte Zone (Z4) umfasst;
wobei der Absorptionskern (130) einen vorderen Teil (130a), der sich zwischen dem vorderen Rand (133) und einer querverlaufenden Schrittlinie des Absorptionskerns (130) erstreckt, und einen hinteren Teil (130b) umfasst, der sich zwischen dem hinteren Rand (134) und der querverlaufenden Schrittlinie des Absorptionskerns (130) erstreckt; wobei die erste (Z1) und zweite Zone (Z2) im vorderen Teil (130a) des Absorptionskerns (130) verlaufen und die dritte (Z3) und vierte Zone (Z4) im hinteren Teil (130b) verlaufen, wobei sich die zweite (Z2) und dritte Zone (Z3) zusammen über eine Länge erstrecken, die mindestens 40 % der Länge des Absorptionskerns (130) entspricht; und
wobei der Absorptionskern (130) mit einer oder mehreren Verstärkungszonen (145) versehen ist, wobei die obere Kernhüllschicht (110) an der unteren Kernhüllschicht (120) befestigt ist und sich mindestens teilweise in die zweite (Z2) und/oder dritte Zone (Z3) erstreckt;
wobei die eine oder die mehreren Verstärkungszonen (145) so angeordnet sind, dass für jeden Bereich in der zweiten (Z2) und/oder dritten Zone (Z3), der eine Breite von 50 % einer Breite des Absorptionskerns (130) und eine Länge von 40 % einer Länge des Absorptionskerns (130) aufweist, mindestens eine Verstärkungszone (145) mindestens teilweise in diesem Bereich liegt.

2. Schwimmwindel (100) nach Anspruch 1, wobei sich die zweite (Z2) und dritte Zone (Z3) zusammen über eine Länge erstrecken, die mindestens 50 % der Länge des Absorptionskerns (130) entspricht.

3. Schwimmwindel (100) nach Anspruch 1 oder 2, wobei sich die zweite (Z2) und dritte Zone (Z3) jeweils über eine Länge erstreckt, die mindestens 20 % der Länge des Absorptionskerns (130), vorzugsweise mindestens 25 % der Länge des Absorptionskerns (130) entspricht.

4. Schwimmwindel (100) nach einem der vorstehenden Ansprüche, wobei der Absorptionskern (130) zwischen 50 und 140 g/m² Flockenzellstoff und vorzugsweise zwischen 70 und 120 g/m² Flockenzellstoff umfasst.

5. Schwimmwindel (100) nach einem der vorstehenden Ansprüche, wobei sich die eine oder die mehreren Verstärkungszonen (145) von einem Schrittbereich in Richtung des vorderen und/oder hinteren Querrands (133, 134) erstrecken.

6. Schwimmwindel (100) nach einem der vorstehenden Ansprüche, wobei die eine oder die mehreren Verstärkungszonen (145) eine im Wesentlichen durchgehende Befestigung mit einer Breite von mindestens 1 mm, vorzugsweise mindestens 2 mm, mehr bevorzugt mindestens 3 mm und am meisten bevorzugt mindestens 4 mm umfassen.

7. Schwimmwindel (100) nach einem der vorstehenden Ansprüche, wobei die eine oder die mehreren Verstärkungszonen (145) eine unterbrochene Befestigung an einer Vielzahl von Stellen in einem Abstand voneinander umfassen, wobei die unterbrochene Befestigung eine Breite von mindestens 1 mm, vorzugsweise mindestens 2 mm, mehr bevorzugt mindestens 3 mm und am meisten bevorzugt mindestens 4 mm aufweist.

8. Schwimmwindel (100) nach einem der vorstehenden Ansprüche, wobei die eine oder die mehreren Verstärkungszonen (145) mindestens eine erste längliche Verstärkungszone umfassen, die in der zweiten (Z2) und/oder dritten Zone (Z3) verläuft.

9. Schwimmwindel (100) nach einem der vorstehenden Ansprüche, wobei die eine oder die mehreren Verstärkungszonen (145) eine oder mehrere permanente Befestigungszonen sind, wobei die permanenten Befestigungszonen so konfiguriert sind, dass sie bei Nässe haften bleiben, vorzugsweise für mindestens 30 Minuten.

10. Schwimmwindel (100) nach dem vorstehenden Anspruch, wobei die eine oder die mehreren Verstärkungszonen (145) mindestens eine zweite längliche Verstärkungszone umfassen, die in der zweiten (Z2) und/oder dritten Zone (Z3) verläuft.

11. Schwimmwindel (100) nach dem vorstehenden Anspruch, wobei die erste und die zweite längliche Verstärkungszone nebeneinander verlaufen.

12. Schwimmwindel (100) nach Anspruch 10 oder 11, wobei die erste und zweite längliche Verstärkungszone symmetrisch in Bezug auf eine Längsmittellinie des Absorptionskerns (130) angeordnet sind.

13. Schwimmwindel (100) nach einem der Ansprüche 10 bis 12, wobei ein Querabstand zwischen der ersten und zweiten länglichen Verstärkungszone zwischen 10 % und 50 % der Breite des Absorptionskerns (130) beträgt, vorzugsweise zwischen 15 % und 45 % der Breite des Absorptionskerns (130) und mehr bevorzugt zwischen 20 % und 40 % der Breite des Absorptionskerns (130); und/oder
wobei sich die erste längliche Verstärkungszone von einem vorderen Rand der zweiten Zone (Z2) in Richtung der dritten Zone (Z3) erstreckt, und wobei sich die zweite längliche Verstärkungszone von einem hinteren Rand der dritten Zone (Z3) in Richtung der zweiten Zone erstreckt.

14. Schwimmwindel (100) nach einem der Ansprüche 10 bis 13, wobei die erste längliche Verstärkungszone durch mindestens eine verbindende Verstärkungszone mit der zweiten länglichen Verstärkungszone verbunden ist; und/oder
wobei die mindestens eine verbindende Verstärkungszone mindestens eines von Folgendem umfasst:
- eine vordere verbindende Verstärkungszone, die einen vorderen Endabschnitt der ersten länglichen Verstärkungszone mit einem entsprechenden vorderen Endabschnitt der zweiten länglichen Verstärkungszone verbindet;
- eine hintere verbindende Verstärkungszone, die einen hinteren Endabschnitt der ersten länglichen Verstärkungszone mit einem entsprechenden hinteren Endabschnitt der zweiten länglichen Verstärkungszone verbindet; und/oder
wobei sich die mindestens eine verbindende Verstärkungszone im Wesentlichen in einer Querrichtung des Absorptionskerns (130) erstreckt; und/oder
wobei die mindestens eine verbindende Verstärkungszone einen oder mehrere gerade Abschnitte und/oder einen oder mehrere gekrümmte Abschnitte umfasst; und/oder
wobei die erste längliche Verstärkungszone, die zweite längliche Verstärkungszone und/oder die mindestens eine verbindende Verstärkungszone gemeinsam im Wesentlichen eine "U"-Form oder im Wesentlichen eine "V"-Form bilden; und/oder
wobei die erste längliche Verstärkungszone, die zweite längliche Verstärkungszone und die mindestens eine verbindende Verstärkungszone gemeinsam einen im Wesentlichen umschlossenen Bereich bilden; und/oder
wobei der im Wesentlichen umschlossene Bereich im Wesentlichen eine "O"-Form oder im Wesentlichen eine polygonale Form aufweist, wie etwa eine im Wesentlichen rechteckige Form, eine im Wesentlichen dreieckige Form, eine Rautenform, eine im Wesentlichen sechseckige Form; und/oder
wobei die erste längliche Verstärkungszone eine Längsmittellinie des Absorptionskerns (130) an mindestens einem ersten Kreuzungspunkt kreuzt; und die zweite längliche Verstärkungszone die Längsmittellinie an mindestens einem zweiten Kreuzungspunkt kreuzt; wobei der erste und der zweite Kreuzungspunkt derselbe oder ein unterschiedlicher Punkt sind und sich im vorderen Teil und/oder im hinteren Teil des Absorptionskerns (130) befinden.

15. Schwimmwindel (100) nach einem der vorstehenden Ansprüche, wobei sich die erste Zone (Z1) über eine Länge erstreckt, die mindestens 5 %, vorzugsweise mindestens 10 % der Länge des Absorptionskerns (130), in Längsrichtung gesehen, entspricht; und/oder
wobei sich die vierte Zone (Z4) über eine Länge erstreckt, die, in Längsrichtung gesehen, mindestens 10 % der Länge des Absorptionskerns (130) entspricht, vorzugsweise mindestens 20 %; und/oder
wobei sich die zweite (Z2) und/oder dritte Zone (Z3) jeweils über eine Länge erstrecken, die, in Längsrichtung gesehen, mindestens 25 % der Länge des Absorptionskerns (130) entspricht, vorzugsweise mindestens 30 %; und/oder
wobei jede der einen oder mehreren Verstärkungszonen (145) eine Länge aufweist, die größer ist als 5 % der Länge des Absorptionskerns (130), vorzugsweise größer als 10 % der Länge des Absorptionskerns (130), mehr bevorzugt größer als 15 % der Länge des Absorptionskerns (130); und/oder
wobei die eine oder die mehreren Verstärkungszonen (145) zusammen mindestens 30 %, vorzugsweise mindestens 40 %, mehr bevorzugt mindestens 50 % und am meisten bevorzugt mindestens 60 % der Länge des Absorptionskerns (130) bedecken; und/oder
wobei die Befestigung zwischen der oberen Kernhüllschicht (110) und der unteren Kernhüllschicht (120) eine der Folgenden oder eine Kombination davon ist: Druckbindung, Wärmebindung, Schallbindung, chemische Bindung, Klebstoff; und/oder
wobei die eine oder die mehreren Verstärkungszonen (145) eine dritte Verstärkungszone umfassen; wobei vorzugsweise die eine oder die mehreren Verstärkungszonen (145) eine vierte Verstärkungszone umfassen; wobei vorzugsweise die dritte Verstärkungszone und die vierte Verstärkungszone symmetrisch in Bezug auf eine Längsmittellinie des Absorptionskerns (130) angeordnet sind; und/oder
wobei die eine oder die mehreren Verstärkungszonen (145) mindestens teilweise in der ersten Zone verlaufen; und/oder
wobei die eine oder die mehreren Verstärkungszonen (145) mindestens teilweise in der vierten Zone verlaufen; und/oder
wobei die eine oder die mehreren Verstärkungszonen (145) mindestens eine Querverstärkungszone umfassen, die sich in Querrichtung des Absorptionskerns (130) erstreckt; und/oder
wobei die Schwimmwindel (100) eine Schwimmhose ist.

## Revendications

1. Couche de bain (100) comprenant une feuille de dessus perméable aux liquides, une feuille de dessous imperméable aux liquides, et une âme absorbante (130) positionnée entre ladite feuille de dessus et ladite feuille de dessous, ladite âme absorbante ayant un premier et un second bord longitudinal (131, 132) et un bord transversal avant et arrière (133, 134) ;
dans laquelle l'âme absorbante (130) comprend une feuille d'enveloppe d'âme de dessus (110) et une feuille d'enveloppe d'âme de dessous (120) et comprend entre 30 et 160 g/m² de pâte en flocons entre la feuille d'enveloppe d'âme de dessus et la feuille d'enveloppe d'âme de dessous ; dans laquelle l'âme absorbante est sensiblement exempte de polymères superabsorbants ;
dans laquelle, vue dans une direction longitudinale de l'âme absorbante, lorsque l'on regarde depuis le bord transversal avant vers le bord transversal arrière, l'âme absorbante comprend subséquemment une première (Z1), une deuxième (Z2), une troisième (Z3) et une quatrième zone (Z4) ;
dans laquelle l'âme absorbante (130) comprend une partie avant (130a) s'étendant entre le bord avant (133) et une ligne d'entrejambe transversale de l'âme absorbante (130), et une partie arrière (130b) s'étendant entre le bord arrière (134) et la ligne d'entrejambe transversale de l'âme absorbante (130) ; dans laquelle lesdites première (Z1) et deuxième zones (Z2) s'étendent dans la partie avant (130a) de l'âme absorbante (130) et lesdites troisième (Z3) et quatrième zones (Z4) s'étendent dans la partie arrière (130b), dans laquelle les deuxième (Z2) et troisième zones (Z3) s'étendent ensemble sur une longueur correspondant à au moins 40 % de la longueur de l'âme absorbante (130) ; et
dans laquelle l'âme absorbante (130) est pourvue d'une ou plusieurs zones de renforcement (145), où la feuille d'enveloppe d'âme de dessus (110) est attachée à la feuille d'enveloppe d'âme de dessous (120), s'étendant au moins partiellement dans les deuxième (Z2) et/ou troisième zones (Z3) ;
dans lequel les une ou plusieurs zones de renforcement (145) sont agencées de telle sorte que pour l'une quelconque zone dans les deuxième (Z2) et/ou troisième zones (Z3) ayant une largeur de 50 % d'une largeur de l'âme absorbante (130) et une longueur de 40 % d'une longueur de l'âme absorbante (130), au moins une zone de renforcement (145) est au moins partiellement localisée dans ladite zone.

2. Couche de bain (100) selon la revendication 1, dans laquelle les deuxième (Z2) et troisième zones (Z3) s'étendent ensemble sur une longueur correspondant à au moins 50 % de la longueur de l'âme absorbante (130).

3. Couche de bain (100) selon la revendication 1 ou 2, dans laquelle chacune des deuxième (Z2) et troisième zones (Z3) s'étend sur une longueur correspondant à au moins 20 % de la longueur de l'âme absorbante (130), de préférence au moins 25 % de la longueur de l'âme absorbante (130).

4. Couche de bain (100) selon l'une quelconque des revendications précédentes, dans laquelle l'âme absorbante (130) comprend entre 50 et 140 g/m² de pâte en flocons, et de préférence entre 70 et 120 g/m² de pâte en flocons.

5. Couche de bain (100) selon l'une quelconque des revendications précédentes, dans laquelle les une ou plusieurs zones de renforcement (145) s'étendent depuis une région d'entrejambe dans la direction du bord transversal avant et/ou arrière (133, 134).

6. Couche de bain (100) selon l'une quelconque des revendications précédentes, dans laquelle les une ou plusieurs zones de renforcement (145) comprennent une attache sensiblement continue ayant une largeur d'au moins 1 mm, de préférence d'au moins 2 mm, plus préférablement d'au moins 3 mm, et le plus préférablement d'au moins 4 mm.

7. Couche de bain (100) selon l'une quelconque des revendications précédentes, dans laquelle les une ou plusieurs zones de renforcement (145) comprennent une attache discontinue au niveau d'une pluralité de localisations à une distance les unes des autres, l'attache discontinue ayant une largeur d'au moins 1 mm, de préférence d'au moins 2 mm, plus préférablement d'au moins 3 mm, et le plus préférablement d'au moins 4 mm.

8. Couche de bain (100) selon l'une quelconque des revendications précédentes, dans laquelle les une ou plusieurs zones de renforcement (145) comprennent au moins une première zone de renforcement allongée s'étendant dans les deuxième (Z2) et/ou troisième zones (Z3).

9. Couche de bain (100) selon l'une quelconque des revendications précédentes, dans laquelle, les une ou plusieurs zones de renforcement (145) sont une ou plusieurs zones d'attache permanente, lesdites zones d'attache permanente étant conçues pour rester attachées lors de l'humidification, de préférence pendant au moins 30 minutes.

10. Couche de bain (100) selon la revendication précédente, dans laquelle les une ou plusieurs zones de renforcement (145) comprennent au moins une deuxième zone de renforcement allongée s'étendant dans les deuxième (Z2) et/ou troisième zones (Z3).

11. Couche de bain (100) selon la revendication précédente, dans laquelle les première et deuxième zones de renforcement allongées s'étendent l'une à côté de l'autre.

12. Couche de bain (100) selon la revendication 10 ou 11, dans laquelle les première et deuxième zones de renforcement allongées sont agencées symétriquement par rapport à une ligne centrale longitudinale de l'âme absorbante (130).

13. Couche de bain (100) selon l'une quelconque des revendications 10 à 12, dans laquelle une distance transversale entre les première et deuxième zones de renforcement allongées est entre 10 % et 50 % de la largeur de l'âme absorbante (130), de préférence entre 15 % et 45 % de la largeur de l'âme absorbante (130), et plus préférentiellement entre 20 % et 40 % de la largeur de l'âme absorbante (130) ; et/ou
dans laquelle la première zone de renforcement allongée s'étend depuis un bord avant de la deuxième zone (Z2) dans la direction de la troisième zone (Z3), et dans laquelle la deuxième zone de renforcement allongée s'étend depuis un bord arrière de la troisième zone (Z3) dans la direction de la deuxième zone.

14. Couche de bain (100) selon l'une quelconque des revendications 10 à 13, dans laquelle la première zone de renforcement allongée est reliée à la deuxième zone de renforcement allongée à travers au moins une zone de renforcement de liaison ; et/ou
dans laquelle l'au moins une zone de renforcement de liaison comprend au moins l'une parmi :
- une zone de renforcement de liaison avant qui relie une portion d'extrémité avant de la première zone de renforcement allongée à une portion d'extrémité avant correspondante de la deuxième zone de renforcement allongée ;
- une zone de renforcement de liaison arrière qui relie une portion d'extrémité arrière de la première zone de renforcement allongée à une portion d'extrémité arrière correspondante de la deuxième zone de renforcement allongée ; et/ou dans laquelle l'au moins une zone de renforcement de liaison s'étend sensiblement dans une direction transversale de l'âme absorbante (130) ; et/ou
dans laquelle l'au moins une zone de renforcement de liaison comprend une ou plusieurs portions droites, et/ou une ou plusieurs portions incurvées ; et/ou
dans laquelle la première zone de renforcement allongée, la deuxième zone de renforcement allongée, et/ou l'au moins une zone de renforcement de liaison forment collectivement un profil en « U » sensible, ou un profil en « V » sensible ; et/ou
dans laquelle la première zone de renforcement allongée, la deuxième zone de renforcement allongée, et l'au moins une zone de renforcement de liaison forment collectivement une région sensiblement fermée ; et/ou
dans laquelle la région sensiblement fermée a un profil en « O », ou un profil en polygone sensible, telle qu'un profil sensiblement rectangulaire, un profil sensiblement triangulaire, un profil en diamant, un profil sensiblement hexagonal ; et/ou
dans laquelle ladite première zone de renforcement allongée croise une ligne centrale longitudinale de l'âme absorbante (130) en au moins un premier point de croisement ; et ladite deuxième zone de renforcement allongée croise ladite ligne centrale longitudinale en au moins un second point de croisement ; dans laquelle lesdits premier et second points de croisement sont le même point ou un point différent, et sont localisés dans la partie avant et/ou dans la partie arrière de l'âme absorbante (130).

15. Couche de bain (100) selon l'une quelconque des revendications précédentes, dans laquelle la première zone (Z1) s'étend sur une longueur correspondant à au moins 5 %, de préférence au moins 10 % de la longueur de l'âme absorbante (130) vue dans la direction longitudinale ; et/ou
dans laquelle la quatrième zone (Z4) s'étend sur une longueur correspondant à au moins 10 % de la longueur de l'âme absorbante (130) vue dans la direction longitudinale, de préférence au moins 20 % ; et/ou
dans laquelle les deuxième (Z2) et/ou troisième zones (Z3) s'étendent chacune sur une longueur correspondant à au moins 25 % de la longueur de l'âme absorbante (130) vue dans la direction longitudinale, de préférence au moins 30 % ; et/ou
dans laquelle chacune des une ou plusieurs zones de renforcement (145) a une longueur qui est supérieure à 5 % de la longueur de l'âme absorbante (130), de préférence supérieure à 10 % de la longueur de l'âme absorbante (130), plus préférablement supérieure à 15 % de la longueur de l'âme absorbante (130) ; et/ou
dans laquelle les une ou plusieurs zones de renforcement (145) couvrent ensemble au moins 30 %, de préférence au moins 40 %, plus préférablement au moins 50 %, et le plus préférablement au moins 60 % de la longueur de l'âme absorbante (130) ; et/ou
dans laquelle l'attache entre la feuille d'enveloppe de dessus (110) et la feuille d'enveloppe de dessous (120) est l'un quelconque des éléments suivants ou une combinaison de ceux-ci : collage par pression, collage thermique, collage sonique, collage chimique, adhésif ; et/ou
dans laquelle les une ou plusieurs zones de renforcement (145) comprennent une troisième zone de renforcement ; dans laquelle de préférence les une ou plusieurs zones de renforcement (145) comprennent une quatrième zone de renforcement ; dans laquelle de préférence la troisième zone de renforcement et la quatrième zone de renforcement sont agencées symétriquement par rapport à une ligne centrale longitudinale de l'âme absorbante (130) ; et/ou
dans laquelle les une ou plusieurs zones de renforcement (145) s'étendent au moins partiellement dans la première zone ; et/ou
dans laquelle les une ou plusieurs zones de renforcement (145) s'étendent au moins partiellement dans la quatrième zone ; et/ou
dans laquelle les une ou plusieurs zones de renforcement (145) comprennent au moins une zone de renforcement transversale s'étendant dans la direction transversale de l'âme absorbante (130) ; et/ou
dans laquelle la couche de bain (100) est une culotte de bain.
